(19) Europäisches Patentamt
European Patent Office
Office européen des brevets

(11) Publication number: **0 061 341**
**B1**

(12) **EUROPEAN PATENT SPECIFICATION**

(45) Date of publication of patent specification: **30.04.86**

(21) Application number: **82301483.2**

(22) Date of filing: **23.03.82**

(51) Int. Cl.[4]: **C 07 D 471/06,**
C 07 D 221/14, A 61 K 31/47
// C07D217/14, C07D217/16,
(C07D471/06, 231:00,
221:00),(C07D471/06, 221:00,
209:00)

(54) **6-Substituted hexahydroindazolo isoquinolines.**

(30) Priority: **24.03.81 US 247047**

(43) Date of publication of application:
**29.09.82 Bulletin 82/39**

(45) Publication of the grant of the patent:
**30.04.86 Bulletin 86/18**

(84) Designated Contracting States:
**AT BE CH DE FR GB IT LI LU NL SE**

(56) References cited:
**EP-A-0 013 787**
**EP-A-0 013 788**
**EP-A-0 016 274**

(73) Proprietor: **ELI LILLY AND COMPANY**
**307, East McCarty Street**
**Indianapolis Indiana 46285 (US)**

(72) Inventor: **Kornfeld, Edmund Carl**
**5159, East 76th Court**
**Indianapolis Indiana 46250 (US)**
Inventor: **Bach, Nicholas James**
**4269 Burkhart Street East Drive**
**Indianapolis Indiana (US)**
Inventor: **Titus, Robert Daniel**
**1203 North Emerson**
**Indianapolis Indiana 46219 (US)**

(74) Representative: **Crowther, Terence Roger et al**
**Erl Wood Manor**
**Windlesham Surrey GU20 6PH (GB)**

**EP 0 061 341 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention concerns 6-substituted hexahydroindazolo isoquinolines of the formula

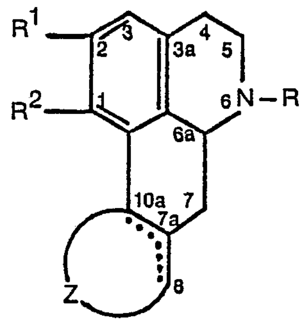

I

wherein

R is H, ($C_1$—$C_3$) alkyl, allyl or benzyl; $R^1$ and $R^2$ are independently H, OH or ($C_1$—$C_3$)alkoxy, with the limitation that when either $R^1$ or $R^2$ is OH, then the other $R^1$ and $R^2$ can not be ($C_1$—$C_3$)alkoxy;

Z is

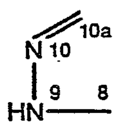 , or 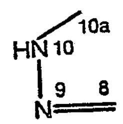 ;

the dotted line represents the presence of one double bond at either 7a-10a or 7a-8; and the acid addition salts thereof.

Compounds according to formula I in which R is H or benzyl are primarily useful as intermediates, while those compounds in which R is ($C_1$—$C_3$)alkyl or allyl are primarily useful in altering the physiological effect of dopamine, either as dopaminergic agents or as dopamine antagonists. Those compounds in which $R^1$ or $R^2$ are ($C_1$—$C_3$)alkoxy are not only useful as dopamine agonists or as dopamine antagonists, but are also useful intermediates in that they can be converted to compounds in which either $R^1$ or $R^2$ or both are OH.

In the above formula the terms ($C_1$—$C_3$)alkyl and($C_1$—$C_3$)alkoxy include for example methyl, methoxy, ethyl, ethoxy, n-propyl, and n-propoxy.

In formula I above, when Z is

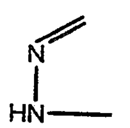 , or 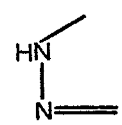 ,

two tautomers have been represented in a conventional way used for depicting three-dimensional molecules in two dimensions. It should be realized, however, that the two pyrazole ring isomers actually exist as a resonance hybrid of the two structures with the negative charge (electron pair) favoring one nitrogen over the other, with the proton located intermediate to the two nitrogens. As will be apparent to those skilled in the art, designating one tautomer automatically designates its companion tautomer since the two exist in dynamic equilibrium.

Pharmaceutically-acceptable acid addition salts of the compounds of formula I include salts derived from non-toxic inorganic acids such as: hydrochloric acid, nitric acid, phosphoric acid, sulfuric acid, hydrobromic acid, hydriodic acid, phosphorous acid and others, as well as salts derived from non-toxic organic acids such as aliphatic mono and dicarboxylic acids, phenyl-substituted alkanoic acids, hydroxy alkanoic and alkandioic acids, aromatic acids, aliphatic and aromatic sulfonic acids. Such pharmaceutically-acceptable salts thus include for example sulfate, pyrosulfate, bisulfate, sulfite, bisulfite, nitrate, phosphate, monohydrogenphosphate, dihydrogenphosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, ß-hydroxybutyrate, glycollate, malate, tartrate, methanesulfonate, propanesulfonate, naphthalene-1-sulfonate, and naphthalene-2-sulfonate.

The following compounds illustrate some of the compounds according to formula I, only the 4,5,6,6a,7,9-hexahydro tautomer has been illustrated, it being understood that the 4,5,6,6a,7,10-tautomer is also named thereby:

1-ethoxy-6-n-propyl-4,5,6,6a,7,9-hexahydroindazolo[7,6,5-ij]isoquinoline maleate
1-methoxy-6-ethyl-4,5,6,6a,7,9-hexahydroindazolo[7,6,5-ij]isoquinoline sulfate
1,2-diethoxy-6-allyl-4,5,6,6a,7,9-hexahydroindazolo[7,6,5-oj]isoquinoline dihydrochloride
6-benzyl-4,5,6,6a,7,9-hexahydroindazolo[7,6,5-ij]isoquinoline lactate
1,2-dimethoxy-4,5,6,6a,7,9-hexahydroindazolo[7,6,5-ij]isoquinoline tartrate
1,2-dihydroxy-6-benzyl-4,5,6,6a-7,9-hexahydroindazolo[7,6,5-ij]isoquinoline dihydrogen phosphate
2-hydroxy-6-n-propyl-4,5,6,6a-7,9-hexahydroindazolo[7,6,5-ij]isoquinoline dinitrobenzoate.

The compounds of formula I above are prepared by reacting an 8-dimethylaminomethylene derivative of the formula

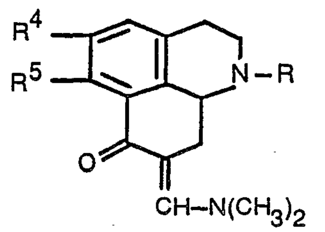 II

wherein
R is defined as above;
$R^4$ and $R^5$ are independently H or ($C_1$—$C_3$)alkoxy; with

$$R^6—NH_2$$

where $R^6$ is $NH_2$—, in a non-reactive solvent;
optionally followed by hydrolysis when $R^3$ is

$$-\overset{\overset{\displaystyle O}{\|}}{C}-(C_1-C_2)\text{alkyl}$$

to obtain the compounds of formula I where $R^3$ is H;
optionally followed by dealkylating the compounds where $R^1$ and/or $R^2$ are ($C_1$—$C_3$)alkoxy to obtain the compounds of formula I wherein $R^1$ and/or $R^2$ are OH; and
optionally followed by reductive alkylation when R is H to obtain the compounds where R is ($C_1$—$C_3$)alkyl or allyl.

The compounds of formula I above result in the formation of 6-substituted hexahydroindazolo isoquinoline products. These products are prepared by reacting a compound of formula II above with anhydrous hydrazine in a non-reactive solvent. Examples of some suitable solvents are $C_1$—$C_6$ alcohols, *i.e.* methanol, ethanol, chloroform, methylenedichloride, ethers, especially diethyl ether, benzene and toluene. The temperature range to run this reaction is from about 0—100°C, with room temperature being preferred.

The compounds of formula II above are claimed in copending European application No. 84201377.3 (EP—A—151319) and are prepared by reacting a suitably 1-substituted 7-oxo-2,3,7,8,9,9a-hexahydro-1H-benzo[de]quinoline of the formula

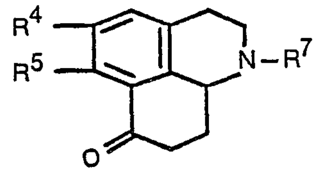 III

wherein:
$R^4$, $R^5$ and $R^7$ are defined as above, with dimethylformamide acetal, tris(dimethylamino)methane, or bis(dimethylamino)methoxymethane.

EP 16274 discloses a group of 2-azaergolines and 2-aza-8(or 9)-ergolenes useful as neuraleptic agents.

The starting 1-substituted-7-oxo-benzo[de]quinolines, formula III, are prepared by somewhat different routes depending on whether $R^4$ is H or alkoxy. For example, Schnieder et al. *Helv. Chim. Acta. 56,* 759 (1973) have prepared 5,6-dimethoxy-7-oxo-2,3,7,8,9,9a-hexahydro-1H-benzo[de]quinoline (formula III above where $R^4$ and $R^5$ are methoxy and R is methyl) by cyclizing N-(3,4-dimethoxyphenylethyl) ß-(methoxycarbonyl)propionamide to yield methyl ß-(6,7-dimethoxy-3,4-dihydro-1-isoquinolinyl)propionate.

Quaternization with RX wherein X is a halogen and R has its previous meaning followed by borohydride reduction of the quaternary salt and hydrolysis of the reduction product gives ß-(6,7-dimethoxy-1,2,3,4-tetrahydro-1-isoquinolyl)propionic acid, cyclization of which with pyrophosphoric acid, oleum or the like yields the desired 7-oxo derivative.

Where there is no activation of the benzene ring ortho to a side chain as in a 3,4-dimethoxy ethylamine, as in the Schnieder et al procedure, a somewhat different synthetic route is used. In this procedure, 1-methylisoquinoline is condensed with chloral to yield, after purification, 1-(3,3,3-trichloro-2-hydroxypropyl)isoquinoline of the formula

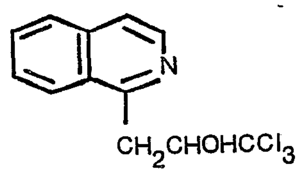

VI

Treatment of VI with base, as for example, an aqueous alkali metal hydroxide, serves both to hydrolyze the trichloromethyl group to a carboxylic acid and to dehydrate the resulting molecule to yield a ß-(1-isoquinolinyl)acrylic acid of the formula

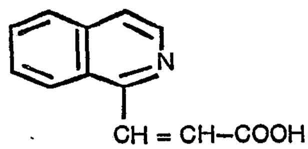

VII

Next, the acrylic acid group of VII is esterified via the acid chloride as with a lower alkanol. The resulting acrylate ester group is then hydrogenated, to a propionate group (formula VIII) preferably with Raney nickel although other catalysts such as Adams catalyst ($PtO_2$) may also be used. Moderate hydrogen pressures (3,515—4,218 g/cm$^2$) are employed and the acrylate ester diluted with a lower alkanol, preferably the same alkanol used to esterify the acrylic acid.

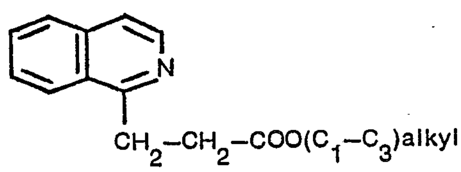

VIII

Next, the ring nitrogen is quaternized, as with methyl, ethyl, n-propyl or allyl iodide, or benzyl bromide or methylfluorosulfonate and the quaternary salt reduced with a metal hydride reducing agent which does not also reduce the ester group. Ordinarily, sodium borohydride is used. The product of this series of reactions is a ß-(1,2,3,4-tetrahydro-1-isoquinolinyl)propionate (IX), wherein in $R^7$ is ($C_1$—$C_3$)alkyl, allyl or benzyl

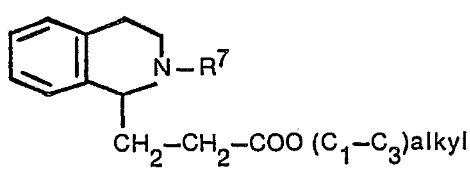

IX

Hydrolysis of the propionate group to the free acid yields a ß-[1-methyl (ethyl, propyl, allyl, benzyl) 1,2,3,4-tetrahydro-1-isoquinolinyl] propionic acid (Va) usually obtained as an acid addition salt such as a hydrochloride salt.

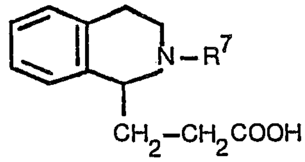

Va

A cyclization procedure, using oleum, $P_2O_5$ plus methanesulfonic acid, polyphosphoric acid or the like as the strong acid cyclizing agent, yields the desired intermediate 1-methyl(ethyl, propyl, allyl, benzyl)-7-oxo-2,3,7,8,9,9a-hexahydro-1H-benzo[de]quinoline (IV) (III when $R^4$ and $R^5$ are H and $R^7$ is $C_1$—$C_3$alkyl, allyl or benzyl).

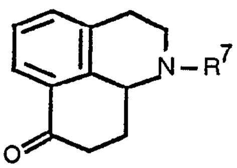

IV

When $R^7$ is methyl, the above two steps in the synthetic procedure can be reversed, i.e., the ß-(1-isoquinolinyl)acrylic acid VII can be esterified and the acrylate ester quaternized as with methylfluorosulfonate and the methyl quaternary salt reduced with borohydride to yield an acrylate ester of structure

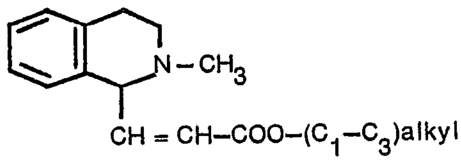

X

Hydrogenation of the acrylate double bond over Raney nickel followed by hydrolysis of the resulting propionate ester yields a propionic acid derivative (Va wherein $R^7$ is $CH_3$). Cyclization of this derivative yields a tricyclic ketone (IV wherein $R^7$ is $CH_3$) produced by an alternate pathway.

If quaternization in either procedure is carried out with a benzyl halide (or pseudo halide), the final product of the reaction sequence will be a 6-benzyl-4,5,6,6a,7,9-hexahydroindazolo[7,6,5-ij]isoquinoline and its 4,5,6,6a,7,10-hexahydro tautomer. The benzyl group can then be removed by catalytic hydrogenation according to standard procedures to produce those intermediates of this invention of formula I in which R is H, which intermediates can be reductively alkylated, for example with formaldehyde, acetaldehyde, propionaldehyde or acrylaldehyde and sodium cyanoborohydride in a lower alkanol such as methanol to produce the corresponding N-methyl, N-ethyl, N-n-propyl or N-allyl compounds of this invention (I in which R = $(C_1—C_3)$alkyl or allyl). Catalytic hydrogenation can also be used.

In formula II, when $R^4$ and $R^5$ are both $(C_1—C_3)$alkoxy or one is $(C_1—C_3)$alkoxy and the other H, the alkoxy group can be dealkylated by well known methods, e.g. 48% HBr in glacial acetic acid, to yield the corresponding OH group for $R^1$ and/or $R^2$ in formula I.

Compounds according to formula I as well as the 3-ring intermediates II, III, and IV contain an asymmetric center-carbon 7a in formula I and carbon 9a in formulas II, III or IV. These compounds represented by the above formulas exist in two diastereoisomeric forms. In the ordinary synthetic procedure, the two stereoisomers are produced in equal amounts and the product is isolated as a dl pair or racemate. The racemate can be separated into its component stereoisomers by methods available in the art, or by salt formation with an optically active acid. It is not known whether each stereoisomer will have some degree of physiologic activity or whether all such activity will reside in a single isomer. This invention covers isomers possessing dopaminergic or dopamine antagonist activity or both. Racemic mixtures of a physiologically active isomer with an inactive isomer are useful in providing the pharmacologic affect of the active isomer without the time and expense of an isomer separation.

Also provided by this invention are pharmaceutical formulations which comprise as active ingredient a 6-substituted hexahydroindazolo or hexahydroisoindolo isoquinoline of formula I or the pharmaceutically acceptable acid addition salt thereof, as well as a method for affecting the physiological role of dopamine which comprises administering to a mammal an effective amount of the pharmaceutical formulation.

This invention is further illustrated by the following specific examples. The nmr data was run on a 100 mHz machine in $CDCl_3$.

Example 1

Preparation of 6-Methyl-4,5,6,6a,7,9-hexahydroindazolo[7,6,5-ij]isoquinoline and 6-Methyl-4,5,6,6a,7,10-hexahydroindazolo[7,6,5-ij]isoquinoline

A reaction mixture was prepared by dissolving 50 g. of 1-methylisoquinoline, 40 g. pyridine and 70 g. of chloral in 400 ml. of dioxane. The reaction mixture was heated to reflux temperature under a nitrogen atmosphere for 2.75 hours and was then allowed to remain overnight at ambient temperature. Evaporation of the volatile constituents left a residue which was dissolved in ether. The ethereal solution was filtered through Florisil® and the filtrate saturated with gaseous hydrogen chloride. 1-(3,3,3-trichloro-2-hydroxypropyl)isoquinoline hydrochloride, being insoluble in ether, precipitated and the precipitate was collected by filtration. Recrystallization of the filter cake from methanol-ether yielded crystals melting with decomposition at about 217°C.

| | | | | |
|---|---|---|---|---|
| Analysis Calculated: | C, 44.07; | H, 3.39; | N, 4.28; | Cl, 43.36 |
| Found: | C, 44.31; | H, 3.12; | N, 4.02; | Cl, 43.38 |

In a second run based on 15 g. of starting 1-methylisoquinoline, there were obtained 29.28 g. of 1-(3,3,3-trichloro-2-hydroxypropyl)isoquinoline hydrochloride melting with decomposition at about 210°C.

Next, a solution was prepared containing 100 ml. of 50% aqueous sodium hydroxide, 50 ml. of water and 150 ml. of ethanol. To this solution was added 10 g. of 1-(3,3,3-trichloro-2-hydroxypropyl)isoquinoline

hydrochloride. The reaction mixture was kept at about 50°C during the addition. After the addition had been completed, the reaction mixture was stirred at room temperature under a nitrogen atmosphere overnight. The reaction mixture was then cooled and made acid by the addition of 12N aqueous hydrochloric acid to a pH of about 6. The resulting solution was extracted several times with a mixture of chloroform and isopropanol. The organic extracts were separated and combined and dried. Evaporation of the dried solution *in vacuo* yielded a solid residue comprising ß-(1-isoquinolinyl)acrylic acid formed in the above reaction. Recrystallization of the residue from a mixture of methanol and chloroform yielded crystals melting at about 172—174°C with vigorous decomposition.

| | |
|---|---|
| Analysis Calculated: | C, 72.35; H, 4.55; N, 7.03 |
| Found: | C, 72.14; H, 4.56; N, 6.71 |

In a second run using 10 g. of 1-(3,3,3-trichloro-2-hydroxypropyl)isoquinoline hydrochloride, about 3.5 g. of the acrylic acid were obtained.

Three and one-half grams of ß-(1-isoquinolinyl)acrylic acid were dissolved in about 250 ml. of methanol and the resulting solution cooled in an ice-water mixture. 2 ml. of thionyl chloride were added in dropwise fashion. The reaction mixture was stirred with cooling for about an hour and then at room temperature for about four days. The volatile constituents were removed by evaporation *in vacuo* and the reaction mixture diluted with aqueous sodium bicarbonate. The aqueous layer was extracted with chloroform, the chloroform layer separated and washed with saturated aqueous sodium chloride and then dried. Evaporation of the chloroform yielded methyl ß-(1-isoquinolinyl)acrylate formed in the above reaction. The residue was suspended in ether and the resulting suspension chromatographed over 150 g. of florisil. Ether was used as the eluant. Fractions shown TLC to contain the desired methyl ester were collected and the solvent removed therefrom by evaporation *in vacuo.* Recrystallization of the solid ester from an ether-hexane solvent mixture yielded methyl ß-(1-isoquinolinyl)acrylate melting at 57—9°C; yield = 895 mg.

| | |
|---|---|
| Analysis Calculated | C, 73.23; H, 5.20; N, 6.57 |
| Found: | C, 72.97; H, 5.06; N, 6.28 |

In an alternative procedure in which the intermediates were not isolated, 22 g. of 1-(3,3,3-trichloro-2-hydroxypropyl)isoquinoline hydrochloride were added to a solution of 200 ml. of 50% aqueous sodium hydroxide, 100 ml. of water and 300 ml. of ethanol held at 25°C. with cooling. The reaction mixture was stirred overnight under a nitrogen atmosphere; then cooled and acidified to a pH of about 2. The volatile constituents were removed by evaporation *in vacuo.* The solid residue was triturated with methanol and filtered. The filtrate was diluted with 350 ml. of methanol and cooled. 15 ml. of thionyl chloride were thereto added in dropwise fashion. The subsequent reaction mixture was stirred at ambient temperature overnight and then diluted with water. The aqueous mixture was made basic with 14N aqueous ammonium hydroxide and the resulting alkaline solution extracted several times with ethyl acetate. The ethyl acetate extracts were separated and combined and the combined extracts washed with saturated aqueous sodium chloride and were then dried. Evaporation of the solvent yielded 6.20 g. of methyl ß-(1-isoquinolinyl)acrylate.

A reaction mixture was prepared containing 24.5 g. of methyl β-(1-isoquinolinyl)acrylate, 15 g. of methyl fluorosulfonate and 400 ml. of methylenedichloride. The reaction mixture was heated to refluxing temperature under a nitrogen atmosphere for about 16 hours whereupon it was cooled and the volatile constituents removed *in vacuo.* The residue, comprising the fluorosulfonate salt of methyl ß-(2-methyl-1-isoquinolinyl)acrylate formed in the above reaction, was dissolved in about 800 ml. of methanol. 25 g. of sodium borohydride were added to this solution in small portions. After the reaction had gone to completion, the reaction mixture was diluted with water and the aqueous mixture extracted several times with ethyl acetate. The ethyl acetate extracts were separated and combined and the combined extracts washed with saturated aqueous sodium chloride and then dried. Evaporation of the ethyl acetate yielded 22.5 g. of methyl ß-(2-methyl-1,2,3,4-tetrahydro-1-isoquinolinyl)acrylate formed in the above reaction.

In another run in which the isoquinolinylacrylate ester was quaternized with methyl iodide in acetonitrile, borohydride reduction yielded 1.03 g. of methyl ß-(2-methyl-1,2,3,4-tetrahydro-1-isoquinolinyl)acrylate.

The hydrochloride salt was prepared by dissolving the oily free base in ether and saturating the ethereal solution with gaseous hydrogen chloride. The hydrochloride salt was recrystallized from a mixture of methanol and ether; melting point = 162—4°C. with decomposition. The salt had the following elemental analysis.

| | |
|---|---|
| Calculated: | C, 62.80; H, 6.78; N, 5.23; Cl. 13.24 |
| Found: | C, 62.58; H, 6.57; N, 5.19; Cl, 13.37 |

0.73 g. of methyl ß-(2-methyl-1,2,3,4-tetrahydro-1-isoquinolinyl)acrylate were hydrogenated over 1 g. of Raney nickel in 98 ml. of methanol at 4218 g/cm.$^2$. The hydrogenation mixture was filtered and the

volatile constituents were removed by evaporation *in vacuo.* The resulting residue was dissolved in ether and the ethereal solution filtered through Florisil. The ethereal filtrate was saturated with gaseous hydrogen chloride and the resulting precipitate crystallized from a mixture of methanol and ether to yield methyl ß-(2-methyl-1,2,3,4-tetrahydro-1-isoquinolinyl)propionate hydrochloride melting at 110—112°C; yield = 40 mg.

| | |
|---|---|
| Analysis Calculated: | C, 62.33; H, 7.47; N, 5.19; Cl, 13.14 |
| Found: | C, 62.59; H, 7.41; N, 5.26; Cl, 13.22 |

A solution was prepared from 1.39 grams of the above ester hydrochloride and 100 ml. of 1N aqueous hydrochloride acid. The resulting acidic solution was heated to refluxing temperature for about 25 hours. The reaction mixture was then cooled and the volatile constituents evaporated *in vacuo.* The resulting residue, comprising ß-(2-methyl-1,2,3,4-tetrahydro-1-isoquinolinyl)propionic acid hydrochloride formed in the above hydrolysis, was triturated with about 250 ml. of boiling acetone. The triturate was filtered and the filtrate concentrated to about 100 ml. and then cooled. 320 mg. of ß-(2-methyl-1,2,3,4-tetrahydro-1-isoquinolinyl)propionic acid hydrochloride thus prepared melted at 147—9°C with decomposition; yield = 320 mg.

| | |
|---|---|
| Analysis Calculated: | C, 61.05; H, 7.09; N, 5.48 |
| Found: | C, 61.19; H, 6.88; N, 5.48 |

In a similar run, using 22.5 g. of the unsaturated ester starting material, Raney nickel reduction followed by hydrolysis of the ester group yielded 13.2 g. of the free propionic acid salt melting at 141—4°C with decomposition. In a third run, 5.3 g. of methyl ß-(2-methyl-1,2,3,4-tetrahydro-1-isoquinolinyl)propionate yielded 4.3 g. of ß-(2-methyl-1,2,3,4-tetrahydro-1-isoquinolinyl)propionic acid hydrochloride melting at 144—7°C.

Four and three-tenths grams of ß-(2-methyl-1,2,3,4-tetrahydro-1-isoquinolinyl)propionic acid hydrochloride were added in small portions to 80 ml. of 18M aqueous sulfuric acid plus 20 ml. of fuming sulfuric acid (about 65% $SO_3$). This mixture was heated in the range of 85—90°C for about 2.5 hours under a nitrogen atmosphere. The mixture was then poured onto ice and the resulting aqueous solution made basic by the addition of 50% aqueous sodium hydroxide. The resulting alkaline solution was extracted several times with equal volumes of methylene dichloride. The methylene dichloride extracts were combined and the combined extracts washed with saturated aqueous sodium chloride. The combined extracts were dried and the solvent removed therefrom by evaporation *in vacuo* to yield 2.60 g. of amorphous 1-methyl-2,3,7,8,9,9a-hexahydro-7-oxo-1H-benzo[de]quinoline formed in the above reaction.

In a second run, 2.6 g. of starting material yielded 0.37 g. of free base.

The free base was dissolved in ether and the ethereal solution saturated with gaseous hydrogen chloride to yield the corresponding hydrochloride salt. After recrystallizing from a methanol-ether solvent mixture, 1-methyl-7-oxo-2,3,7,8,9,9a-hexahydro-1H-benzo[de]quinoline hydrochloride was obtained in essentially quantative yield, melting at about 275°C with decomposition.

| | |
|---|---|
| Analysis Calculated: | C, 65.68; H, 6.78; N, 5.89; Cl, 14.91 |
| Found: | C, 65.48; H, 6.54; N, 6.08; Cl, 14.64 |

A ring closure procedure utilizing phosphorus pentoxide and methanesulfonic acid yielded 2.0 g. of the 1H-benzo[de]quinoline based upon 2.4 g. of β-(2-methyl-2,3,4-tetrahydro-1-isoquinolinyl)propionic acid hydrochloride.

A mixture of 2 g. of 1-methyl-7-oxo-2,3,7,8,9,9a-hexahydro-1H-benzo[de]quinoline and 50 g. of dimethylformamide acetal were refluxed under a nitrogen atmosphere for about 15.5 hours. The reaction mixture was cooled and the volatile constituents removed by evaporation *in vacuo.* A solution of the residue was filtered through Florisil. Evaporation of the solvent yielded 1.9 g. of a compound showing only one major spot and no starting material by TLC. Recrystallization of this same product obtained in a similar run from an ether-hexane solvent mixture yielded 620 mg. of 1-methyl-7-oxo-8-dimethylaminomethylene-2,3,7,8,9,9a-hexahydro-1H-benzo[de]quinoline. m.p. 99—101°C;

| nmr: (δ) | H's | multiplicity | Group |
|---|---|---|---|
| 7.8—8.0 | 2 | m | aryl-H and —C=C—H |
| 7.2—7.4 | 2 | m | aryl-H |
| 3.2 | 6 | s | N—$(CH_3)_2$ (8 position) |
| 2.5 | 3 | s | N—$CH_3$ (1 position) |

| | |
|---|---|
| Analysis Calculated: | C, 74.97; H, 7.86; N, 10.93 |
| Found: | C, 74.74; H, 7.93; N, 10.64 |

3.2 g. of 1-methyl-7-oxo-8-dimethylaminomethylene-2,3,7,8,9,9a-hexahydro-1H-benzo[de]quinoline were dissolved in 100 ml. of methanol. Two milliliters of anhydrous hydrazine were added and the resulting

mixture stirred under a nitrogen atmosphere at ambient temperature for about 24 hours. The reaction mixture was then heated to reflux under a nitrogen atmosphere for about 4 hours after which time it was cooled and the solvents evaporated *in vacuo.* The resulting residue was dissolved in chloroform and the chloroform solution chromatographed over 35 g. of Florisil using chloroform containing increasing amounts (2—5%) of methanol as the eluant. Fractions shown by TLC to contain a single substance were combined. Fractions shown to contain more than one substance were also combined and rechromatographed. 2.0 g. of purified amorphous 6-methyl-4,5,6,6a,7,9-hexahydroindazolo-[7,6,5-ij]isoquinoline and its tautomer, 6-methyl-4,5,6,6a,7,10-hexahydroindazolo[7,6,5-ij]isoquinoline formed in the above reaction were obtained. The product was amorphous. The hydrochloride salt of the tautomeric mixture was prepared by dissolving the amorphous free base in ethanol and adding 0.7 ml. of 12N aqueous hydrochloric acid thereto. Recrystallization of the solid hydrochloride salt from methanol yielded, essentially quantatively, crystals melting at about 260°C with decomposition.

Analysis Calculated: C, 64.24; H, 6.16; N, 16.05; Cl, 13.54
Found: C, 64.44; H, 6.10; N, 16.26; Cl, 13.58

The dihydrochloride salt was also prepared by using an excess of hydrochloric acid. This salt melted above 240°C with decomposition.

## Example 2

Preparation of 6-n-Propyl-4,5,6,6a,7,9-hexahydroindazolo[7,6,5-ij]isoquinoline and 6-n-Propyl-4,5,6,6a,7,10-hexahydroindazolo[7,6,5-ij]isoquinoline

Following the procedure of Example 1, 22 g. of 1-(3,3,3-trichloro-2-hydroxypropyl)isoquinoline hydrochloride were reacted with concentrated aqueous sodium hydroxide to form ß-(1-isoquinolinyl)acrylic acid. Reaction of the acid with thionyl chloride formed the acid chloride. Reaction of the acid chloride with methanol yielded the corresponding methyl ester in 49% yield. Following the procedure of Example 1, the acrylate ester was hydrogenated over Raney nickel to yield methyl ß-(1-isoquinolinyl)propionate. 18 g. of a yellow oil (80.5% yield) were recovered.

Next the isoquinolinyl nitrogen was quaternized with propyl iodide in acetonitrile as a solvent. The resulting quaternary salt was reduced with sodium borohydride in ethanol to yield methyl ß-(2-n-propyl-1,2,3,4-tetrahydro-1-isoquinolinyl)propionate. 7.7 g. of the starting material yielded 4.2 g. of the tetrahydro derivative (44.9% yield). The corresponding hydrochloride salt was prepared in ethereal solution with gaseous HCl; M.P. = 151—3°C.

Mass spectrum; m/e = 215 (free base)
Analysis calculated: C, 62.28; H, 5.23; N, 5.59; O, 12.76; Cl, 14.14;
Found: C, 62.10; H, 5.40, N, 5.43; O, 12.93; Cl 14.35.

About 4.2 g. of methyl ß-(2-n-propyl-1,2,3,4-tetrahydro-1-isoquinolinyl)propionate were stirred for about 20 hours in the presence of 200 ml. of 1N aqueous hydrochloric acid at ambient temperature under a nitrogen atmosphere. The volatile constituents were removed by evaporation and the glassy residue treated with acetone. A white solid separated comprising ß-(2-n-propyl-1,2,3,4-tetrahydro-1-isoquinolinyl)propionic acid hydrochloride was obtained melting at about 208—209°C; yield = 2.49 g.

A suspension was prepared by adding 5 g. of phosphorous pentoxide to 50 g. of methanesulfonic acid (35 ml.). To this suspension were added 3.5 g. of ß-(2-n-propyl-1,2,3,4-tetrahydro-1-isoquinolinyl)propionic acid hydrochloride. The reaction mixture was heated in an oil bath at a temperature in the range 85—90°C. for about one hour under a nitrogen atmosphere. The reaction mixture was then poured over ice and the consequent aqueous mixture made basic by the addition of 50% aqueous sodium hydroxide. The alkaline mixture was extracted several times with equal volumes of methylene dichloride. The organic extracts were combined and the combined extracts washed with saturated aqueous sodium chloride and then dried. Removal of the solvent *in vacuo* yielded an amorphous residue comprising 1-n-propyl-7-oxo-2,3,7,8,9,9a-hexhydro-1H-benzo[de]quinoline formed in the above reaction. The residue was dissolved in ether and the ethereal solution chromatographed over 50 g. of florisil using ether as the eluant. Fractions containing the desired compound (as shown by TLC) were combined. The solvent was removed from the combined fractions *in vacuo* to yield 1.35 g. (47.5%) of a brown oil. An aliquot of the oily residue was dissolved in ether and the ethereal solution saturated with gaseous hydrogen chloride. 1-n-Propyl-7-oxo-2,3,7,8,9,9a-hexahydro-1H-benzo[de]quinoline hydrochloride thus formed melted above 240°C after recrystallization from a methanol/ether solvent mixture. Mass spectrum, m/e at 229.

Analysis Calculated: C, 67.79; H, 7.59; N, 5.27; O, 6.02; Cl, 13.34;
Found: C, 67.51; H, 7.60; N, 5.19; O, 6.27; Cl, 13.53

Thin layer chromatography, (9:1 chloroform/methanol, single spot) $R_f$ = 0.80.

Following the procedure of Example 1, 1.2 g. of 1-n-propyl-7-oxo-2,3,7,8,9,9a-hexahydro-1H-benzo[de]quinoline were dissolved in benzene and the benzene solution treated with 10 g. of tris(dimethylamino)methane (instead of DMF acetal). The reaction was carried out and the desired product purified according to the procedure of Example 1 to yield 1.4 g. of a dark red oil (94.1% yield) comprising 1-n-propyl-7-oxo-8-dimethylaminomethylene-2,3,7,8,9,9a-hexahydro-1H-benzo[de]quinoline formed in the above reaction. Mass spectrum has m/e of 284 for the major peak.

| nmr: (δ) | H's | multiplicity | Group |
|---|---|---|---|
| 3.2 | 6 | s | $—N(CH_3)_2$ |
| 0.9 | 3 | t | $—CH_2CH_2—\underline{CH_3}$ |
| 7.8 | 1 | s | $=C\underline{H}—N$ |

Following the procedure of Example 1, 1.4 g. of 1-n-propyl-7-oxo-8-dimethylaminomethylene-2,3,7,8,9,9a-hexahydro-1H-benzo[de]quinoline were dissolved in methanol and the methanolic solution treated with anhydrous hydrazine to yield an amorphous tautomeric mixture of 6-n-propyl-4,5,6,6a,7,9-hexahydroindazolo[7,6,5-ij]isoquinoline and 6-n-propyl-4,5,6,6a,7,10-hexahydroindazolo[7,6,5-ij)isoquinoline. The tautomeric mixture was isolated as the maleate salt which was prepared by adding an ethereal solution of maleic acid to an ethereal solution of the free base. The maleate salt melted at 178—80°C with decomposition after repeated recrystallization from an ether-methanol solvent mixture. Yield = .360 g.

TLC (9:1 chloroform/methanol) $R_f$ = .58
Analysis Calculated: C, 65.03; H, 6.28; N, 11.37; O, 17.32
Found: C, 65.18; H, 6.43; N, 11.11; O, 17.06
Mass spectrum, m/e at 253 (free base).

Example 3

Preparation of 1,2-Dimethoxy-6-benzyl-4,5,6,6a,7,9-hexahydroindazolo[7,6,5-ij]isoquinoline and 1,2-Dimethoxy-6-benzyl-4,5,6,6a,7,10-hexahydroindazolo[7,6,5-ij]isoquinoline

A solution was prepared by dissolving 29.5 g. of methyl N—(3,4-dimethoxyphenyl)ethyl-4-oxo-4-aminobutanoate — prepared by the method of *Helv. Chim. Acta, 56,* 759 (1973) — in 125 ml. of methylenedichloride. 31.3 g. of phosphorus pentachloride were added portionwise while the mixture was maintained at a temperature of about 20°C. The reaction mixture was then poured over about 150 g. of ice and the consequent aqueous mixture made basic by the addition of 50% (w/v) aqueous potassium carbonate. The organic layer was separated and the alkaline aqueous phase extracted twice with 150 ml. portions of methylenedichloride. The methylene dichloride extracts were combined and dried. Evaporation of the methylenedichloride *in vacuo* yielded a residue comprising methyl ß-(6,7-dimethoxy-3,4-dihydro-1-isoquinolinyl)propionate formed in the above reaction. The residue was twice dissolved in benzene and the benzene immediately evaporated therefrom leaving eventually a blue oil (weight = 27.7—30 g.). The oil was dissolved in 250 ml. of acetonitrile. 24 ml. of benzylbromide were added, thus forming a quaternary salt. The reaction mixture was heated to refluxing temperature under nitrogen for about 16 hours after which time it was cooled and the solvent removed therefrom by evaporation. The residue, comprising the benzylbromide quaternary salt of the isoquinoline was dissolved in 250 ml. of ethanol of 5 g. of sodium borohydride added thereto in portions with external cooling supplied. This reduction mixture was stirred for about one hour at ambient temperature after which time the solvent was removed *in vacuo* and the resulting residue dissolved in methylenedichloride. The methylenedichloride mixture was poured into cold 50% aqueous sodium carbonate. The organic layer was separated and the alkaline layer extracted several times with equal portions of methylenedichloride. The methylenedichloride extracts were combined and dried. Removal of the solvent therefrom *in vacuo* yielded a residue comprising methyl ß-(6,7-dimethoxy-2-benzyl-1,2,3,4-tetrahydro-1-isoquinolinyl)propionate. The residue was taken up in 200 ml. of 1N aqueous hydrochloric acid. The resulting solution was decolorized and filtered through supercell. The resulting filtrate was evaporated to dryness *in vacuo.* The resulting residue was dissolved in acetone and the acetone solution poured into ether. A light solid formed which was separated by filtration. The filter cake was washed several times with ether. 19.6 g. (50.1%) of ß-(6,7-dimethoxy-2-benzyl-1,2,3,4-tetrahydro-1-isoquinolinyl)propionic acid hydrochloride melting at 105—108°C were obtained. This material was added to a solution of 57.5 g. of 18M sulfuric acid and 14.7 g. of oleum (60% $SO_3$). The reaction mixture was stirred for about 5 minutes at 85—90°C and then poured into a mixture of 300 g. of ice and 600 ml. of water. The resulting aqueous mixture was made basic with 25% aqueous sodium hydroxide and the resulting alkaline phase extracted several times with equal volumes of methylenedichloride. The methylenedichloride extracts were combined and the combined extracts dried. Evaporation of the solvent *in vacuo* left an oily residue which was dissolved in ether and chromatographed over 150 g. of florisil using ether as the eluant. Fractions shown by TLC to contain 1-benzyl-5,6-dimethoxy-7-oxo-2,3,7,8,9,9a-hexahydro-1H-benzo[de]quinoline formed in the above reaction were combined. The column was then eluted with chloroform and an additional quantity of material was obtained; total yield = 5.2 g. (30.8% yield). An aliquot of the benzo[de]quinoline eluted with ether and shown to be one spot with an $R_f$ = .85 in a 9:1

chloroform/methanol solvent system was dissolved in ether and the ethereal solution was saturated with gaseous hydrogen chloride. The ether was decanted from the solid hydrochloride salt. The salt melted above 205°C after recrystallization from a methanol-ether solvent mixture.

Analysis Calculated: C, 67.46; H, 6.47; N, 3.75; O, 12.84; Cl, 9.48
Found: C, 67.40; H, 6.44; N, 3.53; O, 12.67; Cl, 9.97
Mass spectrum, m/e at 337 (free base).

Following the procedure of Example 2, 4.5 g. of 1-benzyl-5,6-dimethoxy-7-oxo-2,3,7,8,9,9a-hexahydro-1H-benzo[de]quinoline were dissolved in 100 ml. of benzene to which was added 10 g. of tris(dimethylamino)methane. The reaction was carried out and the product isolated as before. 1-benzyl-5,6-dimethoxy-7-oxo-8-dimethylaminomethylene-2,3,7,8,9,9a-hexahydro-1H-benzo[de]quinoline thus prepared was purified by chromatography over 150 g. of Florisil using chloroform containing increasing quantities (up to 2%) of methanol as the eluant. Fractions shown by TLC to contain the desired dimethylaminomethylene derivative were combined and the solvent removed therefrom by evaporation *in vacuo.* The resulting amorphous residue was dissolved in 100 ml. of anhydrous methanol to which were added 3.7 ml. of anhydrous hydrazine. The reaction was carried out and the compound isolated by the procedure of Example 1. The tautomers, 6-benzyl-1,2-dimethoxy-4,5,6,6a,7,9-hexahydroindazolo[7,6,5-ij]isoquinoline and the corresponding 4,5,6,6a,7,10-hexahydro derivative were purified by chromatography over silica gel using chloroform containing increasing quantities (0.5—5%) of methanol as the eluant. Fractions shown by TLC to contain the desired indazoloisoquinoline were combined and the solvents removed therefrom *in vacuo.* The residual glassy oil was recrystalized from the ether/hexane solvent mixture to yield yellowish crystals melting at 153—156°C.

The corresponding maleate salt was prepared in essentially quantative yield by dissolving the free base in methanol and adding an ethereal solution of maleic acid. The maleate salt of 6-benzyl-1,2,-dimethoxy-4,5,6,6a,7,9-hexahydroindazolo[7,6,5-ij]isoquinoline and the corresponding 4,5,6,6a,7,10-hexahydro derivative melted at 173.5—175°C.

| nmr | ($\delta$) | H's | multiplicity | Group |
|---|---|---|---|---|
| | 3.9 | 6 | d | $-OCH_3$ both |
| | 6.5 | 2 | s | $-CH_3-\phi$ |
| | 7.5 | 1 | s | 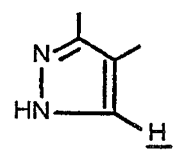 |

The maleate salt had the following analysis.
Calculated: C, 65.90; H, 5.70; N, 8.80;
Found: C, 65.63; H, 5.89; N, 8.53.
Mass spectrum, m/e at 361 (free base).

## Example 4

Preparation of 1,2-Dimethoxy-6-methyl-4,5,6,6a,7,9-hexahydroindazolo[7,6,5-ij]isoquinoline and 1,2-Dimethoxy-6-methyl-4,5,6,6a,7,10-hexahydroindazolo[7,6,5-ij]isoquinoline.

Thirty grams of methyl ß-(6,7-dimethoxy-3,4-dihydro-1-isoquinolinyl)propionate prepared by the procedure of *Helv. Chim. Acta., 56,* 759 (1973) were dissolved in 125 ml. of toluene. 28.4 g. of methyl iodide were added and the mixture heated to refluxing temperature for about 5 hours. The toluene was separated from the quaternized dihydroisoquinoline by decantation and any residual toluene removed by evaporation *in vacuo.* The resulting dark blue residue was dissolved in 200 ml. of ethanol to which were added 8.2 g. of sodium cyanoborohydride portionwise. External cooling was supplied. The reaction mixture remained at ambient temperature for about one-half hour after which time methylenedichloride was added and the resulting mixture poured over a mixture of 50 g. of ice and 50 ml. of 50% aqueous potassium carbonate. The organic layer was separated and the aqueous layer extracted three times with 100 ml. portions of methylenedichloride. The methylenedichloride extracts were combined and the combined extracts dried. The solvent was removed therefrom by evaporation *in vacuo.* The resulting residue comprising methyl ß-(6,7-dimethoxy-2-methyl-1,2,3,4-tetrahydro-1-isoquinolinyl)propionate

formed in the above reaction was dissolved in 200 ml. of 1N aqueous hydrochloric acid. The resulting solution was decolorized with carbon and filtered through supercell. Evaporation of the filtrate to dryness yielded an oily residue. Upon addition of acetone, the residue crystalized yielding a finely divided white solid; weight = 14.5 g. ß-(6,7-dimethoxy-2-methyl-1,2,3,4-tetrahydro-1-isoquinolinyl)propionic acid hydrochloride thus prepared melted 235—237°C.

Following the procedure of Example 3, β-(6,7-dimethoxy -2 - methyl - 1,2,3,4 - tetrahydro - 1 - isoquinolinyl) - propionic acid hydrochloride was cyclized with fuming sulfuric acid to give 1 - methyl - 5,6 - dimethoxy - 7 - oxo - 2,3,7,8,9,9a - hexahydro - 1H - benzo[de]quinoline. 5.3 g. of a faintly yellow transparent oil comprising the tricyclic ketone were obtained from 8.6 g. of the propionic acid derivative. This product is described by Schneider et al., *Helv. Chim. Acta, 56* 759 (1973).

Following the procedure of Example 1, 6.2 g. of 1 - methyl - 5,6 - dimethoxy - 7 - oxo - 2,3,7,8,9,9a - hexahydro - 1H - benzo[de]quinoline were reacted with 50 ml. of DMF acetal. The reaction was carried out and the product isolated by the procedure of Example 1. Chromatography of the isolated product over florisil using chloroform containing increasing amounts (up to 1%) of methanol yielded 4.1 g. of a yellow solid comprising 1 - methyl - 5,6 - dimethoxy - 7 - oxo - 8 - dimethylaminomethylene - 2,3,7,8,9,9a - hexahydro - 1H - benzo[de]quinoline formed in the above reaction. m.p. 160—162°C.

| nmr | (δ) | H's | multiplicity | Group |
|---|---|---|---|---|
| | 3.2 | 6 | s | $-N-(\underline{CH_3})_2$ |
| | 2.6 | 3 | s | $-N-\underline{CH_3}$ (1-position) |
| | 6.8 | 1 | s | 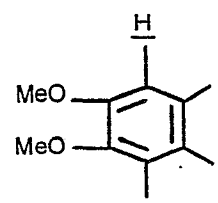 |
| | 7.8 | 1 | s | 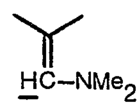 |

Treatment of the above dimethylaminomethylene derivative with anhydrous hydrazine by the procedure of Example 1 yielded 2.2 g. (from 4.1 g. of starting material) of 1,2-dimethoxy-6-methyl-4,5,6,6a,7,9-hexahydroindazolo[7,6,5-ij]isoquinoline dihydrochloride and its 4,5,6,6a,7,10-hexahydro tautomer dihydrochloride, melting with decomposition at about 230°C. The dihydrochloride salts were converted to the free bases by standard procedures. The free bases were dissolved in ether to which was added a saturdated solution of maleic acid. The maleate salts of 1,2-dimethoxy-6-methyl-4,5,6,6a,7,9-hexahydroindazolo[7,6,5-ij]isoquinoline and the corresponding 4,5,6,6a,7,10-hexahydro tautomer melted at about 192.5—194°C.

| NMR | ($\delta$) | H's | multiplicity | Group |
|---|---|---|---|---|
| | 2.6 | 3 | s | N–$CH_3$ |
| | 3.9 | 6 | d | –$OCH_3$ (both) |
| | 6.6 | 1 | s | 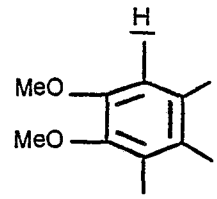 |
| | 7.5 | 1 | s | 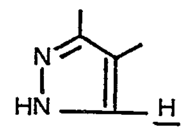 |

Analysis Calculated: C, 59.84; H, 5.78; N, 10.47; O, 23.91
Found: C, 59.65; H, 5.52; N, 10.48; O, 23.69
Mass spectrum, m/e at 285 (free base).

Example 5

Preparation of 1,2 - Dihydroxy - 6 - methyl - 4,5,6,6a,7,9 - hexahydroindazolo[7,6,5 - ij]isoquinoline and 1,2 - Dihydroxy - 6 - methyl - 4,5,6,6a,7,10 - hexahydroindazolo - [7,6,5 - ij]isoquinoline

A mixture of 0.8 g. of 1,2-dimethoxy - 6 - methyl - 4,5,6,6a,7,9 - hexahydroindazolo[7,6,5 - ij] - isoquinoline and the corresponding 4,5,6,6a,7,10-hexahydro tautomer and 21 ml. of 48% hydrobromic acid in 7 ml. of glacial acetic acid were heated to about 135°C. for about 3 hours under a nitrogen atmosphere. The reaction mixture was then cooled. A solid, comprising 1,2-dihydroxy-6-methyl-4,5,6,6a,7,9-hexahydroindazolo[7,6,5-ij]isoquinoline and its 4,5,6,6a,7,10-hexahydro tautomer, both as the hydrobromide salts, precipitated and the precipitate was collected by filtration. The filter cake was washed with acetone and then dissolved in ethanol. Ether was added thereto to the point of incipient precipitation. Upon cooling, a tan solid formed which was separated by filtration. The filtrate was evaporated to dryness in vacuo leaving a white solid. The white solid was suspended in ethanol, filtered and dried. 0.42 Grams of 1,2-dihydroxy-6-methyl-4,5,6,6a,7,9-hexahydroindazole[7,6,5-ij]isoquinoline dihydrobromide and the corresponding 4,5,6,6a,7,10-hexahydro tautomer hydrobromide thus prepared in substantially quantative yield melted at about 240°C.

Analysis Calculated: C, 40.12; H, 4.09; N, 10.03
Found: C, 40.37; H, 4.15; N, 10.13

Example 6

Preparation of 1,2-Dimethoxy-6-n-propyl-4,5,6,6a,7,9-hexahydroindazolo(7,6,5-ij]isoquinoline and 1,2-Dimethoxy-6-n-propyl-4,5,6,6a,7,10-hexahydroindazole[7,6,5-ij]isoquinoline.

Following the procedure of Example 4, 27.7 g. of methyl β-[1-(6,7-dimethoxy-3,4-dihydro-isoquinolinyl)]-propionate were quarternized with n-propyl iodide. The quarternary salt was in turn reduced with sodium borohydride following the procedure of Example 4 and the methyl ester thus obtained hydrolyzed to yield β-[1-(6,7-dimethoxy-2n-propyl-1,2,3,4-tetrahydroisoquinolinyl)]-propionic acid hydrochloride melting at about 172—174°C. after recrystallization from acetone. The propionic acid hydrochloride was cyclized with oleum to yield 1-n-propyl-5,6-dimethoxy-7-oxo-2,3,7,8,9,9a-hexahydro-1H-benzo[de]quinoline. Yield = 79.7%. Treatment of the benzo[de]quinoline free base with methanolic hydrochloric acid yielded corresponding hydrochloride salt which melted with decomposition at 206—207°C. after recrystallization from acetone.

Analysis Calculated: C, 62.67; H, 7.42; N, 4.30; O, 14.73; Cl, 10.88
Found: C, 62.41; H, 7.68; N, 4.37; O, 14.70; Cl, 11.11
Mass spectrum, m/e at 289 (free base).

Following the procedure of Example 2, 1.5 g. of the above 7-oxobenzo[de]quinoline (as the free base) were reacted with 1.3 g. of bis(dimethylamino)methoxymethane to yield 1-n-propyl-5,6-dimethoxy-7-oxo-8-dimethylaminomethylene-2,3,7,8,9,9a-hexahydro-1H-benzo[de]quinoline yield = 1.4 g.

Thin layer chromatography (9:1 chloroform/methanol): $R_f$ = .65.

Mass spectrum, m/e at 344.

Following the procedure of Example 1, 1.4 g. of the above 7-oxo-8-dimethylaminomethylene derivative were reacted with 5 ml. of anhydrous hydrazine. A tautomeric mixture of 1,2-dimethoxy-6-n-propyl-4,5,6,6a,7,9-hexahydroindazolo[7,6,5-ij]isoquinoline and 1,2-dimethoxy-6-n-propyl-4,5,6,6a,7,10-hexahydroindazolo[7,6,5-ij]isoquinoline formed in the above reaction were purified by chromatography over Florisil using chloroform containing increasing amounts (up to 2%) of methanol as the eluant. Fractions shown to contain the desired tautomeric mixture were combined and the solvent removed by evaporation *in vacuo.* The resulting residue was dissolved in ether to which was added a saturated ethereal solution of maleic acid, thus forming the maleate salts. 0.4 Gram of light yellow solid were obtained which melted at 187.5—188°C. after recrystallization from an acetone-methanol solvent mixture.

Analysis calculated: C, 61.53; H, 6.43; N, 9.78
Found: C, 61.29; H, 6.65; N, 9.46
Mass spectrum, m/e at 313 (free base).

## Example 7

Preparation of 1,2-Dihydroxy-6-n-propyl-4,5,6,6a,7,9-hexahydroindazolo[7,6,5-ij]isoquinoline and 1,2-Dihydroxy-6-n-propyl-4,5,6,6a,7,10-hexahydroindazolo[7,6,5-ij]isoquinoline.

Following the procedure of Example 5, 1,2-dimethoxy-6-n-propyl-4,5,6,6a,7,9-hexahydroindazolo-[7,6,5-ij]isoquinoline and its 4,5,6,6a,7,10-hexahydro tautomer were demethylated by treatment with 48% aqueous hydrobromic acid. The resulting tautomeric mixture was purified by the method of Example 5 to yield 1,2-dihydroxy-6-n-propyl-4,5,6,6a,7,9-hexahydroindazolo[7,6,5-ij]isoquinoline and 1,2-dihydroxy-6-n-propyl-4,5,6,6a,7,10-hexahydroindazolo[7,6,5-ij]isoquinoline as the dihydrobromide salts, melting at 234—6°C. with decomposition; 51.6% yield of first crop material. Recrystallization was from an ether-ethanol solvent mixture.

Analysis Calculated: C, 41.40; H, 4.86; N, 9.66; O, 7.35; Br, 36.72
Found: C, 41.26; H, 5.00; N, 9.43; O, 7.60; Br, 36.46

## Example 8

Alternate Preparation of 1,2-Dimethoxy-6-methyl-4,5,6,6a,7,9-hexahydroindazolo[7,6,5-ij]isoquinoline and 1,2-Dimethoxy-6-methyl-4,5,6,6a,7,10-hexahydroinadzolo[7,6,5-ij]isoquinoline

One gram of a tautomeric mixture of 1,2-dimethoxy-6-benzyl-4,5,6,6a,7,9-hexahydroindazolo[7,6,5-ij]-isoquinoline and 1,2-dimethoxy-6-benzyl-4,5,6,6a,7,10-hexahydroindazolo[7,6,5-ij]isoquinoline as the maleate salts was dissolved in ethanol. The solution was hydrogenated over a palladium catalyst at 4.2 kg./cm.$^2$ hydrogen in order the remove the 6-benzyl group. After the theoretical quantity of hydrogen had been absorbed, the hydrogenation mixture was removed from the apparatus and the catalyst separated by filtration. The filtered catalyst was washed with hot methanol. The resulting filtrate was evaporated to dryness *in vacuo* to leave a reddish-brown glassy residue. The residue was dissolved in methanol and ether was added to the point of incipient precipitation. Crystals began to appear and the mixture was held at −15°C. overnight. The crystals were separated by filtration and the filter cake washed with ether. Recrystallization yeilded 1,2-dimethoxy-4,5,6,6a,7,9-hexahydroindazolo[7,6,5-ij]isoquinoline and its 4,5,6,6a,7,10-hexahydro tautomer as an off white solid.

TLC (9:1 chloroform/methanol) $R_f$ = .25
m.p. = above 215°C. (decomposition)
Mass spectrum, m/e = 271.

| nmr | (δ) | H's | multiplicity | Group |
|---|---|---|---|---|
| | 3.9 | 6 | d | $-OCH_3$ (both) |
| | 6.6 | 1 | s | 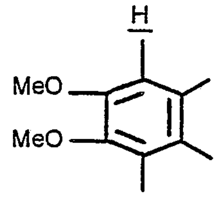 |
| | 7.4 | 1 | s | 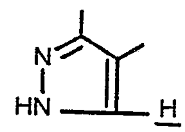 |

During the debenzylation, the maleate salt was reduced to the succinate salt and the compound was isolated and a succinate salt (probably a half succinate salt). The salt was converted to the free base by suspending the solid salt in water and adding 50% aqueous potassium carbonate until the solution was basic. The free base was extracted with chloroform. The chloroform extracts were combined and dried and the chloroform evaporated therefrom. 0.5 Gram of a solid residue was recovered. This residue was dissolved in 20 ml. of methanol to which was added 6.2 ml. of 37% aqueous formaldehyde and 0.75 g. of sodium cyanoborohydride. The reaction mixture was stirred at ambient temperature for about 48 hours, after which time it was poured into 1N aqueous hydrochloric acid. The acidic solution was extracted with an equal volume of ether and the ether extract discarded. The acidic phase was then made basic by the addition of 50% aqueous potassium carbonate. The now alkaline phase was extracted several times with equal volumes of chloroform. The chloroform extracts were combined and dried. Evaporation of the chloroform *in vacuo* left a residue of about 0.2 g. of a yellow viscous oil which showed a single spot on TLC (9:1 $CHCl_3$/MeOH); $R_f$ = .47. The product was identical with 1,2-dimethoxy-6-methyl-4,5,6,6a,7,9-hexahydroindazolo[7,6,5-ij]isoquinoline and its 4,5,6,6a,7,10-hexahydrotautomer as prepared in Example 4.

The free base was converted to the maleate salt which gave yellow crystals melting at about 195—196°C. The nmr of this product was identical with the nmr of the maleate salt of Example 4.

Analysis Calculated: C, 59.84; H, 5.78; N, 10.47
Found: C, 59.55; H, 5.99; N, 10.57

Example 9

Preparation of 1,2-Dimethoxy-6-ethyl-4,5,6,6a,7,9-hexahydroindazolo[7,6,5-ij]isoquinoline and 1,2-Dimethoxy-6-ethyl-4,5,6,6a,7,10-hexahydroindazolo[7,6,5-ij]isoquinoline

When the procedure of Example 4 was repeated using 13.6 g. of methyl β-(6,7-dimethoxy-3,4-dihydro-1-isoquinolinyl)propionate, 60 ml. of toluene, 15.6 g. of ethyl iodide, 100 ml. of ethanol, 4.1 g. of sodium cyanoborohydride and 200 ml. of 1N hydrochloric acid, there was obtained, after recrystallization with acetone and filtration, 6.5 g. (41%) of a light tan solid, β-(6,7-dimethoxy-2-ethyl-1,2,3,4-tetrahydro-1-isoquinolinyl)-propionic acid hydrochloride.

Analysis Calculated: C, 58.27; H, 7.33; N, 4.25; O 19.40
Found: C, 54.56; H, 7.15; N, 4.13; O, 20.85

Following the procedure of Example 3, 5.8 g. of β-(6,7-dimethoxy-2-ethyl-1,2,3,4,-tetrahydro-1-isoquinolinyl)propionic acid hydrochloride was cyclized with fuming sulfuric acid to give 5.1 g. of 1-ethyl-5,6-dimethoxy-7-oxo-2,3,7,8,9,9a-hexahydro-1H-benzo[de]quinoline. After the product was slurried over flurosil, methanol, and filtered, the yield was 3.0 g. (64%). $R_f$ = 0.47.

Following the procedure of Example 1, 3.0 g. of 1-ethyl-5,6-dimethoxy-7-oxo-2,3,7,8,9,9a-hexahydro-1H-benzo[de]quinoline were reacted with 7.2 g. of tris(dimethylamino)methane in 105 ml. of toluene. Chromatography of the isolated product over florisil using 9:1 of methylenedichloride:methanol yielded 3.5 g. (97%), as an orange yellow solid, with $R_f$ = 0.25, of 1-ethyl-5,6-dimethoxy-7-oxo-8-dimethylaminomethylene-2,3,7,8,9,9a-hexahydro-1H-benzo[de]quinoline.

Mass spectrum m/e 330.

| nmr | (δ) | H's | multiplicity | Group |
|---|---|---|---|---|
| | 7.6 | 1 | s | 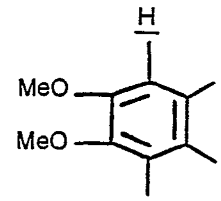 |
| | 6.6 | 1 | s | 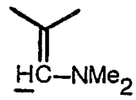 |
| | 3.9 | 6 | d | $-O-CH_3$ (both) |
| | 3.1 | 6 | s | $-N-(CH_3)_2$ |
| | 1.2 | 3 | t | $-CH_2-CH_3$ |

Reaction of the above dimethylaminomethylene derivative, 3.5 g. with 7.4 ml. anhydrous hydrazine by the procedure of Example 1 yielded 2.2 g. (69%) of 1,2-dimethoxy-6-ethyl-4,5,6,6a,7,9-hexahydroindazolo-[7,6,5-ij]isoquinoline and its 4,5,6,6a,7,10-hexahydro tautomer.

| nmr | (δ) | H's | multiplicity | Group |
|---|---|---|---|---|
| | 7.4 | 1 | s | 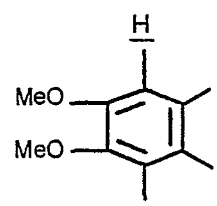 |
| | 6.5 | 1 | s | 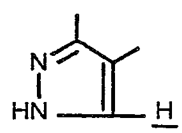 |
| | 3.9 | 6 | d | $-O-CH_3$ (both) |
| | 1.2 | 3 | t | $-CH_2-CH_3$ |

The product was dissolved in hot methanol and saturated maleic acid added. The white precipitate which formed was filtered and washed with ether to yield 2.4 g. of the maleic salts of 1,2-dimethoxy-6-ethyl-4,5,6,6a,7,9-hexahydroindazolo[7,6,5-ij]isoquinoline and the corresponding 4,5,6,6a,7,10-hexahydro tautomer. m.p. 223°C. (decomposition).

Mass spectrum m/e = 298.

Analysis Calculated: C, 60.71; H, 6.07; N, 10.11

Found: C, 60.80; H, 5.99; N, 9.87.

Example 10

Preparation of 1,2-Dimethoxy-6-allyl-4,5,6,6a,7,9-hexahydroindazolo[7,6,5-ij]isoquinoline and 1,2-Dimethoxy-6-allyl-4,5,6,6a,7,10-hexahydroindazolo[7,6,5-ij]isoquinoline

3,4-Dimethoxy-1-(methoxycarbonylethylcarbonylaminoethyl)benzene, 5.0 g., was dissolved in 15 ml. of methylenedichloride. To the solution was added 5.3 g. of phosphorus pentachloride, in portions with stirring and chilling in an ice bath. Stirring was continued in the ice bath for 15 minutes, then continued at room temperature for 3 hours. The reaction mixture was poured onto ice and 30 ml. of 50% aqueous potassium carbonate. The aqueous layer was extracted three times with methylenedichloride, the organic layers combined, stripped of solvent *in vacuo*, and washed with toluene twice. The resulting green-blue residue was dissolved in 18 ml. of acetonitrile and 5.68 g. of allyl iodide was added. The reaction mixture was refluxed under nitrogen for 16 hours. The volatiles were removed *in vacuo* and the residue taken up in 17 ml. of ethanol. An orange-red precipitate formed, 20 ml. of methanol was added, and then 2.2 g. of sodium cyanoborohydride was added with stirring and chilling in an ice bath. The solid disappeared during the reaction and the stirring continued at room temperature for one hour. The solvents were removed *in vacuo*, then the residue was treated with methylenedichloride, ice and 50% aqueous potassium carbonate. The aqueous layer was extracted with methylenedichloride, dried over sodium sulfate, and evaporated to yield methyl β-(6,7-dimethoxy-2-allyl-1,2,3,4-tetrahydro-1-isoquinolinyl)propionate. Mass spectrum parent peak, m/e = 319.

| | | | |
|---|---|---|---|
| Analysis Calculated: | C, 67.69; | H, 7.89; | N, 4.39 |
| Found: | C, 67.60; | H, 7.64; | N, 4.61 |

Methyl β-(6,7-dimethoxy-2-allyl-1,2,3,4-tetrahydro-1-isoquinolinyl)propionate, 0.0169 moles, was heated with 100 ml. of 1N hydrochloric acid for 30 minutes. The mixture was filtered to leave a brown oil. The oil had water removed under vacuum to yield 6.0 g. of a yellow residue. The residue was heated in acetone and cooled to form a white precipitate which was chilled in an ice bath, filtered, washed with acetone and ether to yield 3.1 g. (54%) of β-(6,7-dimethoxy-2-allyl-1,2,3,4-tetrahydro-1-isoquinolinyl)-propionic acid hydrochloride. Mass spectrum major peak at 232 (free base —$CH_2CH_2CO_2H$)

| | | | |
|---|---|---|---|
| Analysis Calculated: | C, 59.73; | H, 7.08; | N, 4.10 |
| Found: | C, 60.02; | H, 6.98; | N, 4.29. |

Following the procedure of Example 3, 2.5 g. of β-(6,7-dimethoxy-2-allyl-1,2,3,4-tetrahydro-1-isoquinolinyl)propionic acid hydrochloride was cyclized with fuming sulfuric acid to give 2.9 g. (95%) of 1-allyl-5,6-dimethoxy-7-oxo-2,3,7,8,9,9a-hexahydro-1H-benzo[de]quinoline.

| nmr | (δ) | H's | multiplicity | Group |
|---|---|---|---|---|
| | 6.8 | 1 | s | 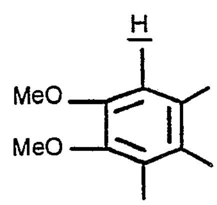 |
| | 3.9 | 6 | s | $-O-CH_3$ (both) |

| | | | |
|---|---|---|---|
| Analysis Calculated: | C, 71.06; | H, 7.37; | N, 4.87 |
| Found: | C, 70.75; | H, 7.48; | N, 5.08. |

A small amount of the above benzo[de]quinoline free base was dissolved in ether and hydrogen chloride gas bubbled into the solution. A white precipitate formed which was filtered, washed with acetone and ether to yield 1-allyl-5,6-dimethoxy-7-oxo-2,3,7,8,9,9a-hexahydro-1H-benzo[de]quinoline hydrochloride, m.p. 186—188°C.

Following the procedure of Example 1, 1.9 g. of 1-allyl-5,6-dimethoxy-7-oxo-2,3,7,8,9,9a-hexahydro-1H-benzo[de]quinoline were reacted with 4.4 g. of tris(dimethylamino)methane in 60 ml. of toluene. Chromatography of the product over Florisil using 9:1 methylenedichloride:methanol yielded 2.1 g. (92%), with $R_f$ = 0.54, of 1-allyl-5,6-dimethoxy-7-oxo-8-dimethylaminomethylene-2,3,7,8,9,9a-hexahydro-1H-benzo[de]quinoline.

| nmr | (δ) | H's | multiplicity | Group |
|---|---|---|---|---|
| | 7.6 | 1 | s | 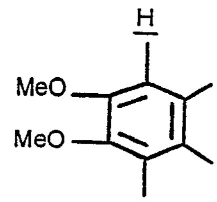 |
| | 6.6 | 1 | s | 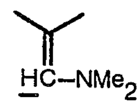 |
| | 3.8 | 6 | d | $-OCH_3$ (both) |
| | 3.0 | 6 | s | $-N(CH_3)_2$ |

Reaction of the above dimethylaminomethylene derivative, 2.1 g. with 4.1 ml. of anhydrous hydrazine by the procedure of Example 1, yielded 360 mg. (19%) of 1,2-dimethoxy-6-allyl-4,5,6,6a,7,9-hexahydroindazolo[7,6,5-ij]isoquinoline and its 4,5,6,6a,7,10-hexahydro tautomer.

| nmr | (δ) | H's | multiplicity | Group |
|---|---|---|---|---|
| | 7.4 | 1 | s | 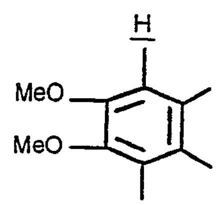 |
| | 6.4 | 1 | s | 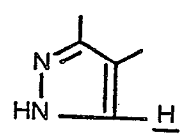 |
| | 3.8 | 6 | d | $-OCH_3$ (both) |

The product was dissolved in ether and a small amount of hot methanol and saturated maleic acid was added. The precipitate which formed was chilled in an ice bath, filtered, and washed with ether to yield 300 mg. of the maleate salts of 1,2-dimethoxy-6-allyl-4,5,6,6a,7,9-hexahydroindazolo[7,6,5-ij]isoquinoline and its 4,5,6,6a,7,10-hexahydro tautomer.

Analysis Calculated: C, 61.82; H, 5.90; N, 9.83
Found: C, 62.09; H, 5.67; N, 9.89.

The compounds of formula I wherein R is ($C_1$—$C_2$)alkyl or allyl affect the physiological role of dopamine in that these compounds affect dopamine receptors. Some receptors are blocked, thus indicating a dopamine antagonist action while at others, the compounds mimic dopamine indicating dopaminergic activity.

Compounds with dopaminergic activity affect turning behaviour in a test procedure utilizing 6-hydroxyopamine-lesioned rats. In this test, nigro-neostriatal-lesioned rats, prepared by the procedure of Ungerstedt and Arbuthnott, *Brain Res, 24,* 485 (1970) are employed. A compound having dopamine agonist activity, upon injection, causes the rats to turn in circles contralateral to the side of the lesion. After a latency period, which varies from compound to compound, the number of turns is counted over a 15-minute period. The drugs to be administered are dissolved in water and the resulting aqueous solution injected into the rat by the intraperitoneal route at a series of dose levels. Table 1 which follows gives the results of such tests. In Table 1, column 1 gives the name of the compound of formula I (one tautomer only is named where required for Z of formula I, but the equilibrium mixture is tested), column 2 the dose, column 3 the percent of rats exhibiting turning behaviour and column 4 the average number of turns.

TABLE 1

Turning Behaviour

| Name of Compound | dose level | Percentage of rats exhibiting turning behaviour | Number of turns |
|---|---|---|---|
| 6-methyl-4,5,6,6a,7,9-hexahydroindazolo[7,6,5-ij]-isoquinoline hydrochloride | 5 mg./kg.<br>1 | 16.6<br>20 | 10<br>16 |
| 6-n-propyl-4,5,6,6a,7,9-hexahydroindazolo[7,6,5-ij]-isoquinoline maleate | 5<br>1 | 70<br>70 | 52.5<br>60.8 |
| 1,2-dihydroxy-6-n-propyl-4,5,6,6a,7,9-hexahydroindazolo-[7,6,5,-ij]isoquinoline dihydro-bromide | 5<br>1 | 100<br>40 | 86.8<br>13.2 |
| 1,2-dimethoxy-6-n-propyl-4,5,6,6a,7,9-hexahydroindazolo-[7,6,5-ij]isoquinoline maleate | 5<br>1 | 40<br>0 | 19.2<br>2.2 |
| 1,2-dihydroxy-6-methyl-4,5,6,6a,7,9-hexahydroindazolo[7,6,5-ij]isoquinoline dihydrobromide | 5<br>1 | 0<br>0 | 0<br>0 |
| 1,2-dimethoxy-6-methyl-4,5,6,6a,7,9-hexahydroindazolo[7,6,5-ij]isoquinoline maleate | 5<br>1 | 0<br>20 | −4<br>0.4 |
| 1,2-dimethoxy-6-ethyl-4,5,6,6a,-7,9-hexahydroindazolo[7,6,5-ij]-isoquinoline maleate | 5<br>1 | 0<br>0 | 0<br>−1.3 |
| 1,2-dimethoxy-6-allyl-4,5,6,6a,-7,9-hexahydroindazolo[7,6,5-ij]-isoquinoline maleate | 5<br>1 | 0<br>0 | 0<br>−1.6 |

One manifestation of the dopamine antagonist activity of the compounds of formula I is their ability to reduce apomorphine-induced turning behaviour in rats. 6-Hydroxydopamine-lesioned rats are prepared as above. The rats are then given apomorphine at a 1 mg./kg. dose and the number of turns and percentage of rats exhibiting turning behaviour measured as before. Next, after the effects of the apomorphine had worn off (2-3 days) rats were predosed with 5 mg./kg. of one of the compounds of formula I and 20 minutes later were given apomorphine at a 1 mg./kg. dose. Percentage of each group exhibiting turning and number of turns in a 15 minute period were measured as before. Table 2 which follows gives the results of these tests. In the table, wherein column 1 gives the name of the compound, columns 2 and 3 percentage of animals exhibiting turning and average number of turns per rats given 1 mg./kg. apomorphine, and columns 4 and 5, same information for rats predosed with a compound of formula I.

TABLE 2
Apomorphine turning behavior

| Name of Compound | Apomorphine alone | | Apomorphine plus drug | |
|---|---|---|---|---|
| | % rats exhibiting turning | Av. No. of turns | % rats exhibiting turning | Av. No. of turns |
| 6-methyl-4,5,6,6a,7,9-hexahydroindazolo[7,6,5-ij]-isoquinoline hydrochloride | 100 | 100.3 | 92 | 67.4 |
| 6-n-propyl-4,5,6,6a,7,9-hexahydroindazolo-[7,6,5-ij]isoquinoline maleate | 100 | 111.3 | 100 | 141.9 |
| 1,2-dihydroxy-6-n-propyl-4,5,6,6a,7,9-hexahydroindazolo[7,6,5-ij]isoquinoline dihydrobromide | 100 | 116.4 | 100 | 116.4 |
| 1,2-dimethoxy-6-n-propyl-4,5,6,6a,7,9-hexahydroindazolo[7,6,5-ij]isoquinoline maleate | 100 | 142 | 100 | 124.4 |
| 1,2-dihydroxy-6-methyl-4,5,6,6a,7,9-hexahydroindazolo[7,6,5-ij]isoquinoline dihydrobromide | 100 | 130.5 | 100 | 121.7 |
| 1,2-dimethoxy-6-methyl-4,5,6,6a,7,9-hexahydroindazolo[7,6,5-ij]isoquinoline maleate | 100 | 142 | 80 | 79.8 |
| 1,2-dimethoxy-6-ethyl-4,5,6,6a,7,9-hexahydroindazolo[7,6,5-ij]isoquinoline maleate | 100 | 104 | 100 | 116 |
| 1,2-dimethoxy-6-allyl-4,5,6,6a,7,9-hexahydroindazolo[7,6,5-ij]isoquinoline maleate | 100 | 127 | 100 | 120 |

The compounds of formula I are also useful as prolactin inhibitors or as prolactin stimulators, acting either as dopaminergic drugs or as dopamine antagonists. Dopaminergic drugs can be employed in the treatment of inappropriate lactation, such as post-partum lactation and galactorrhea. The compounds of formula I have been shown to inhibit or stimulate prolactin according to the following procedure.

Adult male rats of the Sprague-Dawley strain weighing about 200 g. were housed in an air-conditioned room with controlled lighting (lights on 6 a.m.—8 p.m.) and fed lab chow and water *ad libitum*. Each rat received an intraperitoneal injection of 2.0 mg. of reserpine in aqueous suspension 18 hours before administration of the compound of formula I. The purpose of the reserpine was to keep prolactin levels uniformly elevated. The compounds under test were dissolved in water and were injected intraperitoneally at a 1 mg./kg. dose level. Each compound was administered to a group of 10 rats, and a control group of 10 intact males received an equivalent amount of solvent. One hour after treatment all rats were killed by decapitation, and 150 µl. aliquots of serum were assayed for prolactin.

These prolactin levels are given in Table 3 below. In the table, column 1 gives the name of the compound (one tautomer only is named where required for Z of formula I, although the equilibrium mixture was tested) and column 2 the level at a 1 mg./kg. drug dose.

In addition, prolactin levels were determined in two runs in which the rats were not pretreated with reserpine. The dose level of the drug was maintained at 1 mg./kg. Columns 3 and 4 in Table 3 give the prolactin levels found in these two experiments.

TABLE 3
Prolactin Levels

| Name of Compound | Reserpinized | Non-Reserpinized | |
|---|---|---|---|
| | | 1 | 2 |
| 6-methyl-4,5,6,6a,7,9-hexahydroindazolo-[7,6,5-ij]isoquinoline hydrochloride | 39.3±7.3 | 19.7±3.1 | 11.8±2.7 |
| 1,2-dihydroxy-6-methyl-4,5,6,6a,7,9-hexahydroindazolo[7,6,5-ij]isoquinoline dihydrobromide | 36.4±4.9 | 45.2±5.4 (P<.001) | 21.6±3.9 |
| 1,2-dimethoxy-6-methyl-4,5,6,6a,7,9-hexahydroindazolo[7,6,5-ij]isoquinoline maleate | 41.6±7.7 | 36.6±6.6 (P<.02) | 9.0±3.2 |
| 6-n-propyl-4,5,6,6a,7,9-hexahydroindazolo-[7,6,5-ij]isoquinoline maleate | 22.6±3.8 | 9.5±1.7 | 7.8±2.6 |
| 1,2-dihydroxy-6-n-propyl-4,5,6,6a,7,9-hexahydroindazolo[7,6,5-ij]isoquinoline dihydrobromide | 49.9±6.0 (P<.01) | 40.0±9.5 (P<.05) | 23.8±2.8 |
| Control | 27.3±4.2 | 16.9±3.7 | 18.5±9.9 |
| 1,2-dimethoxy-6-ethyl-4,5,6,6a,7,9-hexahydroindazolo[7,6,5-ij]isoquinoline maleate | 30.5±4.9 | 4.6±0.6 | — |
| 1,2-dimethoxy-6-allyl-4,5,6,6a,7,9-hexahydroindazolo[7,6,5-ij]isoquinoline maleate | 26.7±2.7 | 7.3±1.6 | — |
| Control | 27.7±2.8 | 7.0±1.7 | — |

It is apparent from the data presented in Tables 1—3 that the compounds of formula I have unique properties vis-a-vis the various dopamine receptors. Certain compounds affect turning behavior but increase prolactin secretion. Others antagonize apomorphine-induced turning but are without effect by themselves. At least one is predominantly a dopaminergic drug. These results indicate that some of the drugs can act as agonists on dopamine receptors at the striatal level but not in the pituitary. Known antipsychotic drugs stimulate prolactin release. In addition, many neuroleptic drugs have the ability to antagonize apomorphine induced turning in the 6-hydroxy dopamine lesioned rat. Finally, drugs which induced turning in such lesioned rats are useful in the treatment of Parkinson's syndrome and drugs which inhibit prolactin secretion are used to treat inappropriate lactation, as has been set forth above.

In using the dopaminergic compounds of formula I to inhibit prolactin secretion or to treat Parkinson's syndrome or as neuroleptic drugs or for other pharmacological action, a compound according to formula I above, or a salt thereof with a pharmaceutically-acceptable acid, is administered to a subject suffering from Parkinsonism or a phychosis, or in need of having his or her prolactin level reduced, in an amount effective to alleviate some of the symptoms of Parkinsonism or mental disease or to reduce an elevated prolactin level. Oral administration is preferred. If parenteral administration is used, the injection is preferably by the subcutaneous route using an appropriate pharmaceutical formulation. Other modes of parenteral administration such as intraperitoneal, intramuscular, or intravenous routes are equally effective. In particular, with intravenous or intramuscular administration, a water soluble pharmaceutically-acceptable salt is employed. For oral administration, a compound according to formula I, either as the free base or in the form of a salt thereof, is mixed with standard pharmaceutical carriers and the mixture loaded into empty telescoping gelatin capsules or pressed into tablets. The oral dosage should be in the range 0.01—10 mg./kg. of mammalian body weight and the parenteral dose in the range 0.0025 to 2.5 mg./kg.

**Claims for the Contracting States: BE CH DE FR GB IT LI LU NL SE**

1. 6-Substituted hexahydroindazolo isoquinolines of the formula

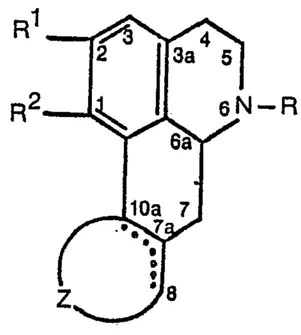

I

wherein

R is H, $(C_1—C_3)$alkyl, allyl or benzyl;

$R^1$ and $R^2$ are independently H, OH or $(C_1—C_3)$alkoxy, with the limitation that when either $R^1$ or $R^2$ is OH, then the other $R^1$ or $R^2$ can not be $(C_1—C_3)$alkoxy;

Z is

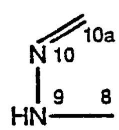
, or
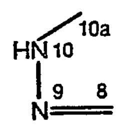
;

the dotted line represents the presence of one double bond at either 7a—10a or 7a—8; and the acid addition salts thereof.

2. A compound of claim 1 wherein R is $(C_1—C_3)$alkyl or allyl and pharmaceutically acceptable acid addition salts thereof.

3. A compound of claim 1 wherein R is H or benzyl.

4. 6-Methyl-4,5,6,6a,7,9-hexahydroindazolo[7,6,5-ij]isoquinoline and its tautomer, 6-methyl-4,5,6,6a,7,10-hexahydroindazolo[7,6,5-ij]isoquinoline.

5. 6-n-Propyl-4,5,6,6a,7,9-hexahydroindazolo[7,6,5-ij]isoquinoline and its tautomer, 6-n-propyl-4,5,6,6a,7,10-hexahydroindazolo[7,6,5-ij]isoquinoline.

6. 1,2-Dimethoxy-6-methyl-4,5,6,6a,7,9-hexahydroindazolo[7,6,5-ij]isoquinoline and its tautomer, 1,2-dimethoxy-6-methyl-4,5,6,6a,7,10-hexahydroindazolo[7,6,5-ij]isoquinoline.

7. 1,2-Dihydroxy-6-methyl-4,5,6,6a,7,9-hexahydroindazolo[7,6,5-ij]isoquinoline and its tautomer, 1,2-dihydroxy-6-methyl-4,5,6,6a,7,10-hexahydroindazolo[7,6,5-ij]isoquinoline.

8. 1,2-Dimethoxy-6-n-propyl-4,5,6,6a,7,9-hexahydroindazolo[7,6,5-ij]isoquinoline and its tautomer, 1,2-dimethoxy-6-n-propyl-4,5,6,6a,7,10-hexahydroindazolo[7,6,5-ij]isoquinoline.

9. 1,2-Dihydroxy-6-n-propyl-4,5,6,6a,7,9-hexahydroindazolo[7,6,5-ij]isoquinoline and its tautomer 1,2-dihydroxy-6-n-propyl-4,5,6,6a,7,10-hexahydroindazolo[7,6,5-ij]isoquinoline.

10. 1,2-Dimethoxy-6-benzyl-4,5,6,6a,7,9-hexahydroindazolo[7,6,5-ij]isoquinoline and its tautomer, 1,2-dimethoxy-6-benzyl-4,5,6,6a,7,10-hexahydroindazolo[7,6,5-ij]isoquinoline.

11. 1,2-Dimethoxy-4,5,6,6a,7,9-hexahydroindazolo[7,6,5-ij]isoquinoline and its tautomer, 1,2-dimethoxy-4,5,6,6a,7,10-hexahydroindazolo[7,6,5-ij]isoquinoline.

12. A pharmaceutical formulation which comprises as active ingredient a 6-substituted hexahydroindazolo isoquinoline of formula I or the pharmaceutically acceptable acid addition salt thereof as claimed in any one of claims 2 or 4—9, and a pharmaceutically acceptable carrier.

13. A 6-substituted hexahydroindazolo isoquinoline of formula I, or a pharmaceutically-acceptable acid addition salt thereof, as claimed in any one of claims 2 or 4—9 for use in affecting the physiological role of dopamine.

14. A process for preparing 6-substituted hexahydroindazolo isoquinolines of the formula I as defined in claim 1 which comprises reacting an 8-dimethylaminomethylene derivative of the formula

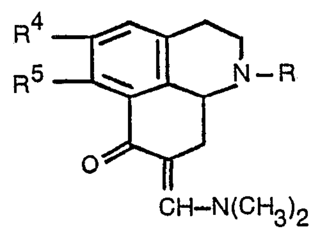

II

wherein
R is defined as above;
$R^4$ and $R^5$ are independently H or ($C_1$—$C_3$)alkoxy; with

$$R^6—NH_2$$

where
$R^6$ is $NH_2$—, in a non-reactive solvent, optionally followed by dealkylating the compounds where $R^1$ and/or $R^2$ are ($C_1$—$C_3$)alkoxy to obtain the compounds of formula I where $R^1$ and/or $R^2$ are OH; and optionally followed by reductive alkylation when R is H to obtain the compounds where R is ($C_1$—$C_3$)alkyl or allyl.

**Claims for the Contracting State: AT**

1. A process for preparing 6-substituted hexahydroindazolo isoquinolines of the formula

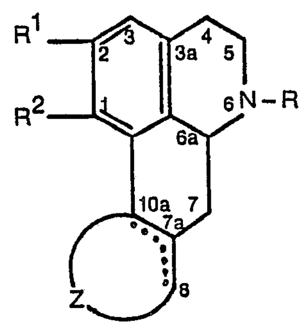

I

wherein
R is H, ($C_1$—$C_3$)alkyl, allyl or benzyl;
$R^1$ and $R^2$ are independently H, OH or ($C_1$—$C_3$)alkoxy, with the limitation that when either $R^1$ or $R^2$ is OH, then the other $R^1$ or $R^2$ can not be ($C_1$—$C_3$)alkoxy;
Z is

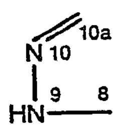

, or

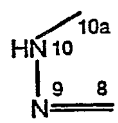

;

the dotted line represents the presence of one double bond at either 7a—10a or 7a—8; and the acid addition salts thereof;
which is characterized by reacting an 8-dimethylaminomethylene derivative of the formula

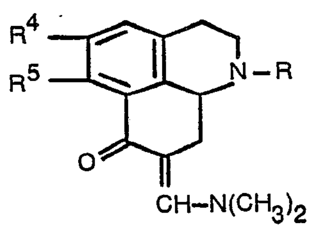

II

wherein
R is defined as above;
$R^4$ and $R^5$ are independently H or ($C_1$—$C_3$)alkoxy; with

$$R^6—NH_2$$

where
$R^6$ is $NH_2$—, in a non-reactive solvent, optionally followed by dealkylating the compounds where $R^1$ and/or $R^2$ are ($C_1$—$C_3$)alkoxy to obtain the compounds of formula I where $R^1$ and/or $R^2$ are OH; and optionally followed by reductive alkylation when R is H to obtain the compounds where R is ($C_1$—$C_3$)alkyl or allyl.

2. A process of claim 1 for preparing the compounds of formula I wherein Z is

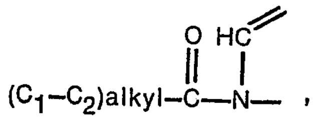 ,

which is characterized by reacting a compound of formula II, as defined in claim 1, with an alkali metal glycinate and then with $(C_1—C_2)$alkyl anhydride.

3. The process of claim 1 for preparing a tautomeric mixture of 6-methyl-4,5,6,6a,7,9-hexahydroindazolo[7,6,5-ij]isoquinoline and 6-methyl-4,5,6,6a,7,10-hexahydroindazolo[7,6,5-ij]isoquinoline which is characterized by reacting 1-methyl-7-oxo-8-dimethyl]aminomethylene-2,3,7,8,9,9a-hexahydro-1H-benzo[de]quinoline with anhydrous hydrazine in methanol.

4. The process of claim 1 for preparing a tautomeric mixture of 6-n-propyl-4,5,6,6a,7,9-hexahydroindazolo[7,6,5-ij]isoquinoline and 6-n-propyl-4,5,6,6a,7,10-hexahydroindazolo[7,6,5-ij]isoquinoline which is characterized by reacting 1-n-propyl-7-oxo-8-dimethylaminomethylene-2,3,7,8,9,9a-hexahydro-1H-benzo[de]quinoline with anhydrous hydrazine in methanol.

5. The process of claim 1 for preparing a tautomeric mixture of 1,2-dimethoxy-6-benzyl-4,5,6,6a,7,9-hexahydroindazolo[7,6,5-ij]isoquinoline and 1,2-dimethoxy-6-benzyl-4,5,6,6a,7,10-hexahydroindazolo-[7,6,5-ij]isoquinoline which is characterized by reacting 1-benzyl-5,6-dimethoxy-7-oxo-8-dimethylaminomethylene-2,3,7,8,9,9a-hexahydro-1H-benzo[de]quinoline with anhydrous hydrazine in methanol.

6. The process of claim 1 for preparing a tautomeric mixture of 1,2-dimethoxy-6-methyl-4,5,6,6a,7,9-hexahydroindazolo[7,6,5-ij]isoquinoline and 1,2-dimethoxy-6-methyl-4,5,6,6a,7,10-hexahydroindazolo-[7,6,5-ij]isoquinoline which is characterized by reacting 1-methyl-5,6-dimethoxy-7-oxo-8-dimethylaminomethylene-2,3,7,8,9,9a-hexahydro-1H-benzo[de]quinoline with anhydrous hydrazine in methanol.

7. The process of claim 1 for preparing a tautomeric mixture of 1,2-dihydroxy-6-methyl-4,5,6,6a,7,9-hexahydroindazolo[7,6,5-ij]isoquinoline and 1,2-dihydroxy-6-methyl-4,5,6,6a,7,10-hexahydroindazolo-[7,6,5-ij]isoquinoline which is characterized by reacting 1-methyl-5,6-dimethoxy-7-oxo-8-dimethylaminomethylene-2,3,7,8,9,9a-hexahydro-1H-benzo[de]quinoline with anhydrous hydrazine in methanol, followed by reacting the product with HBr in glacial acetic acid.

8. The process of claim 1 for preparing a tautomeric mixture of 1,2-dimethoxy-6-n-propyl-4,5,6,6a,7,9-hexahydroindazolo[7,6,5-ij]isoquinoline and 1,2-dimethoxy-6-n-propyl-4,5,6,6a,7,10-hexahydroindazolo-[7,6,5-ij]isoquinoline which is characterized by reacting 1-n-propyl-5,6-dimethoxy-7-oxo-8-dimethylaminomethylene-2,3,7,8,9,9a-hexahydro-1H-benzo[de]quinoline with anhydrous hydrazine in methanol.

9. The process of claim 1 for preparing a tautomeric mixture of 1,2-dihydroxy-6-n-propyl-4,5,6,6a,7,9-hexahydroindazolo[7,6,5-ij]isoquinoline and 1,2-dihydroxy-6-n-propyl-4,5,6,6a,7,10-hexahydroindazolo-[7,6,5-ij]isoquinoline which is characterized by reacting 1-n-propyl-5,6-dimethoxy-7-oxo-8-dimethylaminomethylene-2,3,7,8,9,9a-hexahydro-1H-benzo[de]quinoline with anhydrous hydrazine in methanol, followed by reacting the product with HBr in glacial acetic acid.

**Patentansprüche für die Vertragsstaaten: BE CH DE FR GB IT LI LU NL SE**

1. In Stellung 6 substituierte Hexahydroindazolisochinoline der Formel

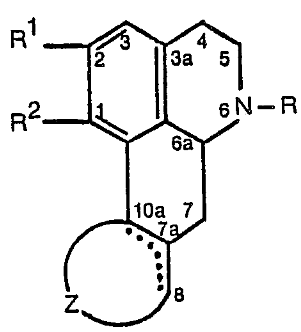 I

worin

R für H, $(C_1—C_3)$-Alkyl, Allyl oder Benzyl steht,

$R^1$ und $R^2$ unabhängig H, OH oder $(C_1—C_3)$-Alkoxy sind, mit der Maßgabe, daß, falls einer der

Substituenten $R^1$ oder $R^2$ für OH steht, der andere der Substituenten $R^1$ oder $R^2$ dann nicht $(C_1—C_3)$-Alkoxy sein kann,

für

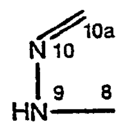 , oder 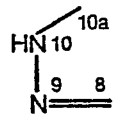 ;

steht und

die punktierte Linie eine Doppelbindung entweder in Stellung 7a—10a oder 7a—8 bedeutet und die Säureadditionssalze hiervon.

2. Verbindung nach Anspruch 1, dadurch gekennzeichnet, daß R für $(C_1—C_3)$-Alkyl oder Allyl steht, und die pharmazeutisch annehmbaren Säureadditionssalze hiervon.

3. Verbindung nach Anspruch 1, dadurch gekennzeichnet, daß R für H oder Benzyl steht.

4. 6-Methyl-4,5,6,6a,7,9-hexahydroindazol[7,6,5-ij]isochinolin und dessen Tautomeres, nämlich 6-Methyl-4,5,6,6a,7,10-hexahydroindazol[7,6,5-ij]isochinolin.

5. 6-n-Propyl-4,5,6,6a,7,9-hexahydroindazol[7,6,5-ij]isochinolin und dessen Tautomeres, nämlich 6-n-Propyl-4,5,6,6a,7,10-hexahydroindazol[7,6,5-ij]isochinolin.

6. 1,2-Dimethoxy-6-methyl-4,5,6,6a,7,9-hexahydroindazol[7,6,5-ij]isochinolin und dessen Tautomeres, nämlich 1,2-Dimethoxy-6-methyl-4,5,6,6a,7,10-hexahydroindazol[7,6,5-ij]isochinolin.

7. 1,2-Dihydroxy-6-methyl-4,5,6,6a,7,9-hexahydroindazol[7,6,5-ij]isochinolin und dessen Tautomeres, nämlich 1,2-Dihydroxy-6-methyl-4,5,6,6a,7,10-hexahydroindazol[7,6,5-ij]isochinolin.

8. 1,2-Dimethoxy-6-n-propyl-4,5,6,6a,7,9-hexahydroindazol[7,6,5-ij]isochinolin und dessen Tautomeres, nämlich 1,2-Dimethoxy-6-n-propyl-4,5,6,6a,7,10-hexahydroindazol[7,6,5-ij]isochinolin.

9. 1,2-Dihydroxy-6-n-propyl-4,5,6,6a,7,9-hexahydroindazol[7,6,5-ij]isochinolin und dessen Tautomeres, nämlich 1,2-Dihydroxy-6-n-propyl-4,5,6,6a,7,10-hexahydroindazol[7,6,5-ij]isochinolin.

10. 1,2-Dimethoxy-6-benzyl-4,5,6,6a,7,9-hexahydroindazol[7,6,5-ij]isochinolin und dessen Tautomeres, nämlich 1,2-Dimethoxy-6-benzyl-4,5,6,6a,7,10-hexahydroindazol[7,6,5-ij]isochinolin.

11. 1,2-Dimethoxy-4,5,6,6a,7,9-hexahydroindazol[7,6,5-ij]isochinolin und dessen Tautomeres, nämlich 1,2-Dimethoxy-4,5,6,6a,7,10-hexahydroindazol[7,6,5-ij]isochinolin.

12. Pharmazeutische Formulierung, dadurch gekennzeichnet, daß sie ein in Stellung 6 substituiertes Hexahydroindazolisochinolin der Formel I oder ein pharmazeutisch annehmbares Säureadditionssalz hiervon gemäß einem der Ansprüche 2 oder 4 bis 9 als Wirkstoff und einen pharmazeutisch annehmbaren Träger enthält.

13. Verwendung eines in Stellung 6 substituierten Hexahydroindazolisochinolins der Formel I oder eines pharmazeutisch annehmbaren Säureadditionssalzes hiervon gemäß einem der Ansprüche 2 oder 4 bis 9 zur Beeinflussung der physiologischen Rolle von Dopamin.

14. Verfahren zur Herstellung von in Stellung 6 substituierten Hexahydroindazolisochinolinen der in Anspruch 1 angegebenen Formel I, dadurch gekennzeichnet, daß man ein 8-Dimethylaminomethylenderivat der Formel

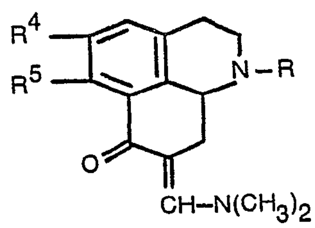

II

worin R wie oben definiert ist,

$R^4$ und $R^5$ unabhängig für H oder $(C_1—C_3)$-Alkoxy stehen, mit

$$R^6—NH_2,$$

worin $R^6$ für $—NH_2$ steht,

in einem nichtreaktionsfähigen Lösungsmittel umsetzt, oder

gegebenenfalls anschließend eine Verbindung, worin $R^1$ und/oder $R^2$ für $(C_1—C_3)$-Alkoxy steht, durch Dealkylierung in eine Verbindung der Formel I überführt, worin $R^1$ und/oder $R^2$ für OH steht, und

gegebenenfalls anschließend eine Verbindung, worin R für H steht, durch reduktive Alkylierung in eine Verbindung überführt, worin R für $(C_1—C_3)$-Alkyl oder Allyl steht.

**Patentansprüche für den Vertragsstaat: AT**

1. Verfahren zur Herstellung von in Stellung 6 substituierten Hexahydroindazolisochinolinen der Formel

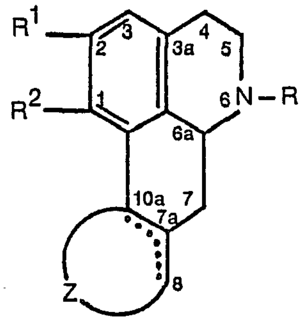

I

worin

R für H, ($C_1$—$C_3$)-Alkyl, Allyl oder Benzyl steht,

$R^1$ und $R^2$ unabhängig H, OH oder ($C_1$—$C_3$)-Alkoxy sind, mit der Maßgabe, daß, falls einer der Substituenten $R^1$ oder $R^2$ für OH steht, der andere der Substituenten $R^1$ oder $R^2$ dann nicht ($C_1$—$C_3$)-Alkoxy sein kann,

Z für

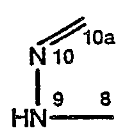 , oder 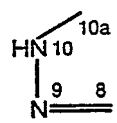 ;

steht und

die punktierte Linie eine Doppelbindung entweder in Stellung 7a—10a oder 7a—8 bedeutet und die Säureadditionssalze hiervon,

dadurch gekennzeichnet, daß man ein 8-Dimethylaminomethylenderivat der Formel

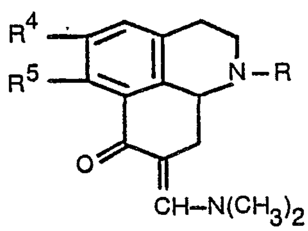

II

worin R wie oben definiert ist,

$R^4$ und $R^5$ unabhängig für H oder ($C_1$—$C_3$)-Alkoxy stehen, mit

$$R^6—NH_2,$$

worin $R^6$ für $—NH_2$ steht,

in einem nichtreaktionsfähigen Lösungsmittel umsetzt, oder

gegebenenfalls anschließend eine Verbindung, worin $R^1$ und/oder $R^2$ für ($C_1$—$C_3$)-Alkoxy steht, durch Dealkylierung in eine verbindung der Formel I überführt, worin $R^1$ und/oder $R^2$ für OH steht, und

gegebenenfalls anschließend eine Verbindung, worin R für H steht, durch reduktive Alkylierung in eine Verbindung überführt, worin R für ($C_1$—$C_3$)-Alkyl oder Allyl steht.

2. Verfahren nach Anspruch 1 zur Herstellung der Verbindungen der Formel I, worin Z für

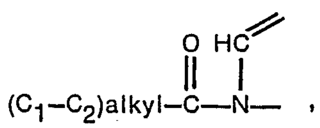 ,

steht, dadurch gekennzeichnet, daß man eine Verbindung der in Anspruch 1 angegebenen Formel II zuerst mit einem Alkalimetallglycinat und dann mit einem ($C_1$—$C_2$)-Alkylanhydrid umsetzt.

3. Verfahren nach Anspruch 1 zur Herstellung eines tautomeren Gemisches aus 6-Methyl-4,5,6,6a,7,9-hexahydroindazol[7,6,5-ij]isochinolin und 6-Methyl-4,5,6,6a,7,10-hexahydroindazol[7,6,5-ij]isochinolin, dadurch gekennzeichnet, daß man 1-Methyl-7-oxo-8-dimethylaminomethylen-2,3,7,8,9,9a-hexahydro-1H-benzo[de]chinolin mit wasserfreiem Hydrazin in Methanol umsetzt.

4. Verfahren nach Anspruch 1 zur Herstellung eines tautomeren Gemisches aus 6-n-Propyl-4,5,6,6a,7,9-hexahydroindazol[7,6,5-ij]isochinolin und 6-n-Propyl-4,5,6,6a,7,10-hexahydroindazol[7,6,5-ij]isochinolin,

dadurch gekennzeichnet, daß man 1-n-Propyl-7-oxo-8-dimethylaminomethylen-2,3,7,8,9,9a-hexahydro-1H-benzo[de]chinolin mit wasserfreiem Hydrazin in Methanol umsetzt.

5. Verfahren nach Anspruch 1 zur Herstellung eines tautomeren Gemisches aus 1,2-Dimethoxy-6-benzyl-4,5,6,6a,7,9-hexahydroindazol[7,6,5-ij]isochinolin und 1,2-Dimethoxy-6-benzyl-4,5,6,6a,7,10-hexahydroindazol[7,6,5-ij]isochinolin, dadurch gekennzeichnet, daß man 1-Benzyl-5,6-dimethoxy-7-oxo-8-dimethylaminomethylen-2,3,7,8,9,9a-hexahydro-1H-benzo[de]chinolin mit wasserfreiem Hydrazin in Methanol umsetzt.

6. Verfahren nach Anspruch 1 zur Herstellung eines tautomeren Gemisches aus 1,2-Dimethoxy-6-methyl-4,5,6,6a,7,9-hexahydroindazol[7,6,5-ij]isochinolin und 1,2-Dimethoxy-6-methyl-4,5,6,6a,7,10-hexahydroindazol[7,6,5-ij]isochinolin, dadurch gekennzeichnet, daß man 1-Methyl-5,6-dimethoxy-7-oxo-8-dimethylaminomethylen-2,3,7,8,9,9a-hexahydro-1H-benzo[de]chinolin mit wasserfreiem Hydrazin in Methanol umsetzt.

7. Verfahren nach Anspruch 1 zur Herstellung eines tautomeren Gemisches aus 1,2-Dihydroxy-6-methyl-4,5,6,6a,7,9-hexahydroindazol[7,6,5-ij]isochinolin und 1,2-Dihydroxy-6-methyl-4,5,6,6a,7,10-hexahydroindazol[7,6,5-ij]isochinolin, dadurch gekennzeichnet, daß man 1-Methyl-5,6-dimethoxy-7-oxo-8-dimethylaminomethylen-2,3,7,8,9,9a-hexahydro-1H-benzo[de]chinolin mit wasserfreiem Hydrazin in Methanol umsetzt und das Produkt dann mit Bromwasserstoff in Eisessig zur Reaktion bringt.

8. Verfahren nach Anspruch 1 zur Herstellung eines tautomeren Gemisches aus 1,2-Dimethoxy-6-n-propyl-4,5,6,6a,7,9-hexahydroindazol[7,6,5-ij]isochinolin und 1,2-Dimethoxy-6-n-propyl-4,5,6,6a,7,10-hexahydroindazol[7,6,5-ij]isochinolin, dadurch gekennzeichnet, daß man 1-n-Propyl-5,6-dimethoxy-7-oxo-8-dimethylaminomethylen-2,3,7,8,9,9a-hexahydro-1H-benzo[de]chinolin mit wasserfreiem Hydrazin in Methanol umsetzt.

9. Verfahren nach Anspruch 1 zur Herstellung eines tautomeren Gemisches aus 1,2-Dihydroxy-6-n-propyl-4,5,6,6a,7,9-hexahydroindazol[7,6,5-ij]isochinolin und 1,2-Dihydroxy-6-n-propyl-4,5,6,6a,7,10-hexahydroindazol[7,6,5-ij]isochinolin, dadurch gekennzeichnet, daß man 1-n-Propyl-5,6-dimethoxy-7-oxo-8-dimethylaminomethylen-2,3,7,8,9,9a-hexahydro-1H-benzo[de]chinolin mit wasserfreiem Hydrazin in Methanol umsetzt und das Produkt dann mit Bromwasserstoff in Eisessig zur Reaktion bringt.

**Revendications pour les Etats contractants: BE CH DE FR GB IT LI LU NL SE**

1. Hexahydroindazolo-isoquinoléines substituées en position 6 et répondant à la formule:

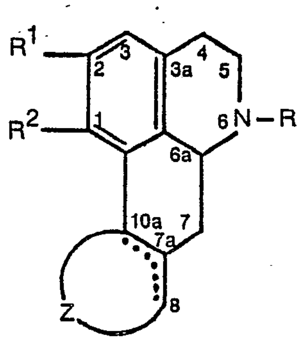

I

dans laquelle

R représente H, un groupe benzyle, un groupe allyle ou un groupe alkyle en $C_1$—$C_3$;

$R^1$ et $R^2$ représentent chacun indépendamment l'un de l'autre H, OH ou un groupe alcoxy en $C_1$—$C_3$, avec cette réserve que, lorsque $R^1$ ou $R^2$ représente OH, l'autre ne peut être un groupe alcoxy en $C_1$—$C_3$;

Z représente

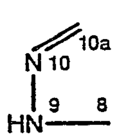 , ou 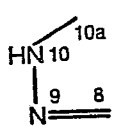 ;

tandis que la ligne en pointillés représente la présence d'une double liaison en position 7a—10a ou 7a—8; ainsi que leurs sels d'addition d'acide.

2. Composé selon la revendication 1, caractérisé en ce que R représente un groupe allyle ou un groupe alkyle en $C_1$—$C_3$, ainsi que ses sels d'addition d'acide pharmaceutiquement acceptables.

3. Composé selon la revendication 1, caractérisé en ce que R représente H ou un groupe benzyle.

4. 6-méthyl-4,5,6,6a,7,9-hexahydroindazolo[7,6,5-ij]isoquinoléne et son tautomère, à savoir la 6-méthyl-4,5,6,6a,7,10-hexahydroindazolo[7,6,5-ij]isoquinoléine.

5. 6-n-propyl-4,5,6,6a,7,9-hexahydroindazolo[7,6,5-ij]isoquinoléine et son tautomère, à savoir la 6-n-propyl-4,5,6,6a,7,10-hexahydroindazolo[7,6,5-ij]isoquinoléine.

6. 1,2-diméthoxy-6-méthyl-4,5,6,6a,7,9-hexahydroindazolo[7,6,5-ij]isoquinoléine et son tautomère, à savoir la 1,2-diméthoxy-6-méthyl-4,5,6,6a,7,10-hexahydroindazolo[7,6,5-ij]isoquinoléine.

7. 1,2-dihydroxy-6-méthyl-4,5,6,6a,7,9-hexahydroindazolo[7,6,5-ij]isoquinolène et son tautomère, à savoir la 1,2-dihyhdroxy-6-méthyl-4,5,6,6a,7,10-hexahydroindazolo[7,6,5-ij]isoquinoléine.

8. 1,2-diméthoxy-6-n-propyl-4,5,6,6a,7,9-hexahydroindazolo[7,6,5-ij]isoquinoléine et son tautomère, à savoir la 1,2-diméthoxy-6-n-propyl-4,5,6,6a,7,10-hexahydroindazolo[7,6,5-ij]isoquinoléine.

9. 1,2-dihydroxy-6-n-propyl-4,5,6,6a,7,9-hexahydroindazolo[7,6,5-ij]isoquinoléine et son tautomère, à savoir la 1,2-dihydroxy-6-n-propyl-4,5,6,6a,7,10-hexahydroindazolo[7,6,5-ij]isoquinoléine.

10. 1,2-diméthoxy-6-benzyl-4,5,6,6a,7,9-hexahydroindazolo[7,6,5-ij]isoquinoléine et son tautomère, à savoir la 1,2-diméthoxy-6-benzyl-4,5,6,6a,7,10-hexahydroindazolo[7,6,5-ij]isoquinoléine.

11. 1,2-diméthoxy-4,5,6,6a,7,9-hexahydroindazolo[7,6,5-ij]isoquinoléine et son tautomère à savoir la 1,2-diméthoxy-4,5,6,6a,7,10-hexahydroindazolo[7,6,5-ij]isoquinoléine.

12. Formulation pharmaceutique comprenant, comme ingrédient actif, une hexahydroindazolo-isoquinoléine substituée en position 6 de formule I ou un de ses sels d'addition d'acide pharmaceutiquement acceptables selon l'une quelconque des revendications 2 ou 4—9, ainsi qu'un support pharmaceutiquement acceptable.

13. Hexahydroindazolo-isoquinoléine substituée en position 6 de formula I ou un de ses sels d'addition d'acide pharmaceutiquement acceptables selon l'une quelconque des revendications 2 ou 4—9 en vue de l'utiliser pour influencer le rôle physiologique de la dopamine.

14. Procédé de préparation d'hexahydroindazolo-isoquinoléines substituées en position 6 de formule I comme défini dans la revendication 1, caractérisé en ce qu'il consiste à faire réagir un dérivé de 8-diméthylaminométhylène de formule:

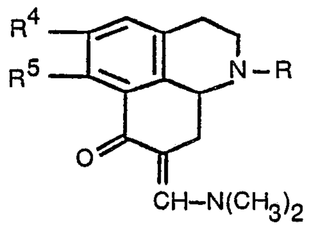

II

dans laquelle

R a la signification définie ci-dessus;

$R^4$ et $R^5$ représentent chacun indépendamment l'un de l'autre H ou un groupe alcoxy en $C_1$—$C_3$;

avec

$$R^6—NH_2$$

où

$R^6$ représente $NH_2$—, dans un solvant non réactif, cette réaction étant éventuellement suivie d'une désalkylation des composés dans lesquels $R^1$ et/ou $R^2$ représentent chacun un groupe alcoxy en $C_1$—$C_3$, pour obtenir les composés de formule I dans laquelle $R^1$ et/ou $R^2$ représentent OH; et cette désalkylation étant facultativement suivie d'une alkylation réductrice lorsque R représente H, pour obtenir les composés dans lesquels R représente un groupe allyle ou un groupe alkyle en $C_1$—$C_3$.

**Revendications pour l'Etat contractant: AT**

1. Procédé de préparation d'hexahydroindazolo-isoquinoléines substituées en position 6 et répondant à la formule:

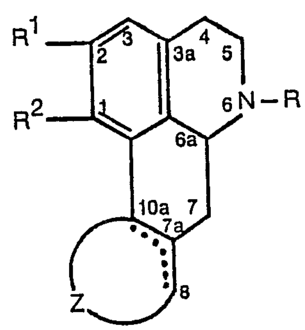

I

dans laquelle

R représente H, un groupe benzyle, un groupe allyle ou un groupe alkyle en $C_1$—$C_3$;

$R^1$ et $R^2$ représentent chacun indépendamment l'un de l'autre H, OH ou un groupe alcoxy en $C_1$—$C_3$,

avec cette réserve que, lorsque $R^1$ ou $R^2$ représente OH, l'autre ne peut être un groupe alcoxy en $C_1$—$C_3$;

Z représente

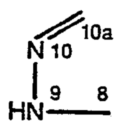 , ou 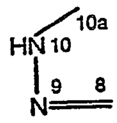 ;

la ligne en pointillés représente la présence d'une double liaison en positon 7a—10a ou 7a—8; ainsi que de leurs sels d'addition d'acide;

caractérisé en ce qu'on fait réagir un dérivé 8-diméthylaminométhylène de formule:

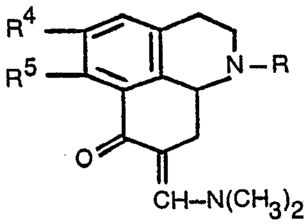 II

dans laquelle

R a la signification définie ci-dessus;

$R^4$ et $R^5$ représentent chacun indépendamment l'une de l'autre H ou un groupe alcoxy en $C_1$—$C_3$; avec

$$R^6\text{—}NH_2$$

où

$R^6$ représente $NH_2$—, dans un solvant non réactif;

cette réaction étant facultativement suivie d'une désalkylation des composés dans lesquels $R^1$ et/ou $R^2$ représentent chacun un groupe alcoxy en $C_1$—$C_3$ pour obtenir les composés de formule I dans laquelle $R^1$ et/ou $R^2$ représentent OH;

et cette désalkylation étant facultativement suivie d'une alkylation réductrice lorsque R représente H, pour obtenir les composés dans lesquels R représente un groupe allyle ou un groupe alkyle en $C_1$—$C_3$.

2. Procédé selon la revendication 1 pour la préparation des composés de formule I dans laquelle Z représente:

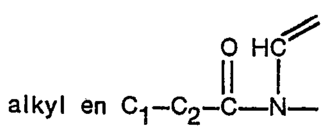

caractérisé en ce qu'on fait réagir un composé de formule II comme défini dans la revendication 1, avec un glycinate d'un métal alcalin, puis avec un anhydride alkylique en $C_1$—$C_2$.

3. Procédé selon la revendication 1 pour la préparation d'un mélange tautomère de 6-méthyl-4,5,6,6a,7,9-hexahydroindazolo[7,6,5-ij]isoquinoléine et de 6-méthyl-4,5,6,6a,7,10-hexahydroindazolo[7,6,5-ij]isoquinoléine, caractérisé en ce qu'il consiste à faire réagir la 1-méthyl-7-oxo-8-diméthylaminométhylène-2,3,7,8,9,9a-hexahydro-1H-benzo[de]quinoléine avec l'hydrazine anhydre dans du méthanol.

4. Procédé selon la revendication 1 pour la préparation d'un mélange tautomère de 6-n-propyl-4,5,6,6a,7,9-hexahydroindazolo[7,6,5-ij]isoquinoléine et de 6-n-propyl-4,5,6,6a,7,10-hexahydroindazolo[7,6,5-ij]isoquinoléine, caractérisé en ce qu'on fait réagir la 1-n-propyl-7-oxo-8-diméthylaminométhylène-2,3,7,8,9,9a-hexahydro-1H-benzo[de]quinoléine avec l'hydrazine anhydre dans du méthanol.

5. Procédé selon la revendication 1 pour la préparation d'un mélange tautomère de 1,2-diméthoxy-6-benzyl-4,5,6,6a,7,9-hexahydroindazolo[7,6,5-ij]isoquinoléine et de 1,2-diméthoxy-6-benzyl-4,5,6,6a,7,10-hexahydroindazolo[7,6,5-ij]isoquinoléine, caractérisé en ce qu'on fait réagir la 1-benzyl-5,6-diméthoxy-7-oxo-8-diméthylaminométhylène-2,3,7,8,9,9a-hexahydro-1H-benzo[de]quinoléine avec l'hydrazine anhydre dans du méthanol.

6. Procédé selon la revendication 1 pour la préparation d'un mélange tautomère de 1,2-diméthoxy-6-méthyl-4,5,6,6a,7,9-hexahydroindazolo[7,6,5-ij]isoquinoléine et de 1,2-diméthoxy-6-méthyl-4,5,6,6a,7,10-hexahydroindazolo[7,6,5-ij]isoquinoléine, caractérisé en ce qu'on fait réagir la 1-méthyl-5,6-diméthoxy-7-oxo-8-diméthylaminométhylène-2,3,7,8,9,9a-hexahydro-1H-benzo[de]quinoléine avec de l'hydrazine anhydre dans du méthanol.

7. Procédé selon la revendication 1 pour la préparation d'un mélange tautomère de 1,2-dihydroxy-6-méthyl-4,5,6,6a,7,9-hexahydroindazolo[7,6,5-ij]isoquinoléine et de 1,2-dihydroxy-6-méthyl-4,5,6,6a,7,10-

hexahydroindazolo[7,6,5-ij]isoquinoléine, caractérisé en ce qu'on fait réagir la 1-méthyl-5,6-diméthoxy-7-oxo-8-diméthylaminométhylène-2,3,7,8,9,9a-hexahydro-1H-benzo[de]quinoléine avec l'hydrazine anhydre dans du méthanol, puis on fait réagir le produit avec HBr dans de l'acide acétique glacial.

8. Procédé selon la revendication 1 pour la préparation d'un mélange tautomère de 1,2-diméthoxy-6-n-propyl-4,5,6,6a,7,9-hexahydroindazolo[7,6,5-ij]isoquinoléine et de 1,2-diméthoxy-6-n-propyl-4,5,6,6a,7,10-hexahydroindazolo[7,6,5-ij]isoquinoléine, caractérisé en ce qu'on fait réagir la 1-n-propyl-5,6-diméthoxy-7-oxo-8-diméthylaminométhylène-2,3,7,8,9,9a-hexahydro-1H-benzo[de]quinoléine avec l'hydrazine anhydre dans du méthanol.

9. Procédé selon la revendication 1 pour la préparation d'un mélange tautomère de 1,2-dihydroxy-6-n-propyl-4,5,6,6a,7,9-hexahydroindazolo[7,6,5-ij]isoquinoléine et de 1,2-dihydroxy-6-n-propyl-4,5,6,6a,7,10-hexahydroindazolo[7,6,5-ij]isoquinoléine, caractérisé en ce qu'on fait réagir la 1-n-propyl-5,6-diméthoxy-7-oxo-8-dimethylaminométhylène-2,3,7,8,9,9a-hexahydro-1H-benzo[de]quinoléine avec l'hydrazine anhydre dans du méthanol, puis on fait réagir le produit avec HBr dans de l'acide acétique glacial.